(19) **Europäisches Patentamt**
**European Patent Office**
**Office européen des brevets**

(11) **EP 3 821 862 B1**

(12) **EUROPEAN PATENT SPECIFICATION**

(45) Date of publication and mention
of the grant of the patent:
**05.07.2023 Bulletin 2023/27**

(21) Application number: **19854299.5**

(22) Date of filing: **04.02.2019**

(51) International Patent Classification (IPC):
**A61F 13/496** *(2006.01)*     **A61F 13/49** *(2006.01)*

(52) Cooperative Patent Classification (CPC):
**A61F 13/4963**

(86) International application number:
**PCT/JP2019/003920**

(87) International publication number:
**WO 2020/044592 (05.03.2020 Gazette 2020/10)**

(54) **ABSORBENT ARTICLE**

ABSORBIERENDER ARTIKEL

ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2018  JP 2018160526**
**26.11.2018  JP 2018220748**

(43) Date of publication of application:
**19.05.2021 Bulletin 2021/20**

(73) Proprietor: **Unicharm Corporation**
**Shikokuchuo-shi, Ehime 799-0111 (JP)**

(72) Inventors:
• **ICHIKAWA, Makoto**
**Kanonji-shi, Kagawa 769-1602 (JP)**
• **TANAKA, Yoshinori**
**Kanonji-shi, Kagawa 769-1602 (JP)**
• **KAWABATA, Kuniyoshi**
**Kanonji-shi, Kagawa 769-1602 (JP)**

(74) Representative: **Dolleymores**
**9 Rickmansworth Road**
**Watford, Hertfordshire WD18 0JU (GB)**

(56) References cited:
EP-A1- 3 545 924     WO-A1-2017/130785
WO-A1-2020/090177     JP-A- 2001 046 432
JP-A- 2004 298 413     JP-A- 2006 192 902

**Description**

TECHNICAL FIELD

The present invention relates to an absorbent article.

BACKGROUND

There has been know an absorbent article (for example, a pants-type disposable diaper) including a stomach-side portion and a back-side portion and having a joining portion in which at least one end part of the stomach-side portion and at least one end part of the back-side portion, both of the at least one end parts being in the lateral direction, are joined to each other along the longitudinal direction in a state of overlapping each other in the thickness direction. As a method for forming the joining portion in the absorbent article, for example, Patent Literature 1 discloses a method in which the joining portion (sealing portion) is formed using a sealing device including an ultrasonic horn and an anvil. In this method, while the conveyance of sheet members for the stomach-side portion and the back-side portion, when predetermined places for the joining portion to be formed in these sheet members pass through the gap between the ultrasonic horn and the anvil, the predetermined places are pinched and fused with the ultrasonic horn and the anvil, thereby forming the joining portion in the predetermined places. In the joining portion, a plurality of fused places aligned along the longitudinal direction, that is, a plurality of fused portions are formed. In addition, Patent Literature 2 discloses another method and a device for forming the joining portion (sealing portion) with an ultrasonic horn and an anvil.

[Patent Literature]

Patent Literature 1: Japanese Unexamined Patent Publication No. 2004-298413
Patent Literature 2: Japanese Unexamined Patent Publication No. 2006-192902

SUMMARY

[Technical Problem]

In the case of removing the above-described absorbent article from a wearer after use, in general, a method is used, in which, in the joining portion, the sheet member in the stomach-side portion and the sheet member in the back-side portion are continuously pulled apart from each other in the longitudinal direction. That is, the sheet member in the stomach-side portion and the sheet member in the back-side portion joined with the plurality of fused portions are continuously pulled apart from each other in the longitudinal direction by breaking the sheet members at each of the fused portions or the circumferential portions thereof. However, in the absorbent article of Patent Literature 1, there may be a case where, in the middle of pulling apart the joining portion, a crack generated in the fused portion or the like proceeds to the sheet member in a portion excluding the joining portion in the stomach-side portion or the back-side portion, for example, the sheet member in the central portion. In such a case, it becomes difficult to continuously pull apart the sheet member in the stomach-side portion and the sheet member in the back-side portion of the joining portion from each other in the longitudinal direction, and additionally, it may be conceivable that an absorbent body may be exposed or excrement may leak through the crack that has proceeded to the central portion. These disadvantages also may apply to the absorbent article of Patent Literature 2 in some cases.
Therefore, an object of the present invention is to provide an absorbent article having a joining portion capable of, at the time of removing the absorbent article, suppressing the breakage of a sheet member in a portion excluding the joining portion and facilitating the appropriate separation of a stomach-side portion and a back-side portion.

[Solution to Problem]

The present invention provides the absorbent article of independent claim 1. The dependent claims specify preferred but optional features.
An absorbent article of the present invention is (1) an absorbent article having a longitudinal direction, a lateral direction, and a thickness direction that are orthogonal to each other, the absorbent article comprising: a stomach-side portion including a stomach-side sheet member; and a back-side portion including a back-side sheet member, wherein the absorbent article further comprises a joining portion in which at least one end part of the stomach-side portion and at least one end part of the back-side portion in the lateral direction are joined to each other along the longitudinal direction in a state of overlapping each other in the thickness direction, wherein the joining portion includes a plurality of fused portions disposed at gaps along the longitudinal direction, wherein each of the plurality of fused portions further includes: a core portion in which the stomach-side sheet member and the back-side sheet member are fused to each other in the thickness direction, and a circumferential wall portion in which the stomach-side sheet member and the back-side sheet member are fused to each other in the thickness direction in a way such that the circumferential wall portion extends from a circumferential edge of the core portion in the thickness direction while surrounding the core portion in a tubular shape, wherein, in a T-type peel test of the joining portion, in which a sample including a part of the joining portion is gripped with two jigs, the joining portion is peeled off such that a gap between the two jigs broadens, and a load that is applied between the two jigs is measured, in a region indicated by a graph showing the load with respect to the gap, when a specific region, in which the load is equal to or greater than a predetermined reference load, is divided into a short-gap side region and a long-gap side region with respect to a maximum load gap as a boundary, the load of which being a maximum value, a ratio between an area a of the short-gap side region and an area b of the long-gap side region, which is represented by a/b, is 0.5 or more and 2.0 or less.

In the present absorbent article, each fused portion in the joining portion has a core portion and a circumferential wall portion. Here, the circumferential wall portion is formed of the stomach-side sheet member and the back-side sheet member that are melted, integrated in the thickness direction, and solidified in a tubular wall shape. Therefore, usually, the circumferential wall portion is strong and does not easily break compared with the stomach-side sheet member and the back-side sheet member in the circumference of the circumferential wall portion. Therefore, in the case of peeling the joining portion, the circumferential wall portion itself does not easily break, and the stomach-side sheet member or the back-side sheet member in the circumference of the circumferential wall portion is likely to break at a boundary portion with the circumferential wall portion or the circumferential portion of the boundary portion (hereinafter, simply referred to as "boundary portion"). Therefore, when the joining portion is pulled apart to remove the absorbent article, it is possible to make breakage likely to occur at the boundary portion with (the circumferential wall portion of) the fused portion in the stomach-side sheet member or the back-side sheet member. As a result, it is possible to make it easy, for example, to leave the fused portion in the stomach-side sheet member and to form a hole due to the removal of the fused portion in the back-side sheet member. Therefore, it is possible to continuously and easily pull apart the portion of the stomach-side portion and the portion of the back-side portion in the joining portion from each other in the longitudinal direction. Here, in a case where the width (in the longitudinal direction or the lateral direction) of the circumferential wall portion is uneven, for example, in a case where the width is partially small, there is a possibility that breakage is likely to occur not at the boundary portion with the fused portion (circumferential wall portion) in the stomach-side sheet member or the back-side sheet member, but in the circumferential wall portion itself (a portion having a small width). When the number of such fused portions (circumferential wall portions) is relatively small, there is a low possibility that breakage may actually occur in the circumferential wall portion itself. However, when the number becomes relatively large, there is a high possibility that breakage may actually occur in the circumferential wall portion itself. In such a case, it becomes difficult to continuously pull apart the portion of the stomach-side portion and the portion of the back-side portion in the joining portion from each other in the longitudinal direction. Therefore, in the present absorbent article, in order to improve the evenness in the width of the circumferential wall portion, in the test of the peel strength of the joining portion, an effort is made in a manner such that a/b that is the ratio of the area a of the short-gap side region and the area b of the long-gap side region becomes 0.5 or more and 2.0 or less. That is, an effort is made in a manner such that the area a of the short-gap side region and the area b of the long-gap side region become substantially the same as each other. Here, since the sheet members are joined to each other in the joining portion mainly with the fused portions, a main factor that affects the peel strength of the joining portion is the sheet members in (the circumferential wall portions of) the fused portions and the circumferences of the fused portions. That is, what appears in the graph showing the relationship between the gap and the load in the peel test can be said to be the load relating to the breakage of the sheet members in (the circumferential wall portions of) the fused portions and the circumferences of the fused portions. Therefore, the short-gap side region indicates the breakage of the sheet member in half of the fused portions on the front side in the peeling direction of the sample with the tester and the circumference of the fused portions (hereinafter, also referred to as "front half portion"). On the other hand, the long-gap side region indicates the breakage of the sheet member in half of the fused portions on the back side in the peeling direction and the circumference of the fused portions (hereinafter, also referred to as "back half portion"). In the present absorbent article, in the specific region indicated by the graph of the peel strength described above, since the area a of the short-gap side region and the area b of the long-gap side region are substantially the same as each other, the energy for the breakage of the front half portion and the energy for the breakage of the back half portion are substantially the same as each other. Therefore, the breakage of the front half portion and the breakage of the back half portion are considered to proceed in substantially the same manner. That is, the state of breakage between the half of the fused portions on the front side and the sheet member in the circumference of the above-described fused portions and the state of breakage between the half of the fused portions on the back side and the sheet member in the circumference of the above-described fused portions are considered to be substantially the same as each other. Therefore, it can be said that, in the circumferential wall portion, a place where the width becomes partially too small is not generated. In other words, it can be said that, in the circumferential wall portion, there are only a small number of places where the width is uneven. As described above, in the absorbent article, the width of the circumferential wall portion is relatively even as a whole in the fused portions. Therefore, when the stomach-side portion and the back-side portion are pulled apart from each other, it is possible to suppress the circumferential wall portion itself being broken due to a force exerted to pull apart the stomach-side portion and the back-side portion, and it is possible to make breakage likely to occur at the boundary portion with the fused portions (circumferential wall portion) in the stomach-side sheet member or the back-side sheet member. That is, it is possible to relatively decrease the number of fused portions that break in the circumferential wall portion. As a result, it is possible to easily pull apart each joining portion into the stomach-side portion and the back-side portion along the longitudinal direction all the way to the end, and it is possible to suppress a phenomenon in which the circumferential wall portion breaks, the breakage proceeds from the broken portion, and the sheet member in a portion excluding the joining portion breaks. As described above, in the present absorbent article having the joining portion, it becomes possible to facilitate, at the time of removing the absorbent article, the appropriate separation of the joining portion into the stomach-side portion and the back-side portion without breaking the sheet member in a portion excluding the joining portion. It should be noted that the predetermined reference load is a load necessary to pull apart the stomach-side sheet member and the back-side sheet member in a portion excluding the fused portions in the sample and is a load relating to the peeling of joining in a portion excluding the fused portions (for example, natural entanglement between fibers or the like). Such a load is, for example, 4 N/25 mm.

The absorbent article of the present invention is (2) the absorbent article according to (1), wherein breaking strengths in the fused portions are higher than breaking strengths of portions not including the fused portions in any one of the stomach-side sheet member and the back-side sheet member. In the present absorbent article, when the stomach-side portion and the back-side portion are pulled apart from each other, it is possible to make breakage more likely to occur at the boundary portion with the fused portion in the sheet member (not including the fused portion) and to suppress the circumferential wall portions breaking at a larger number of the fused portions. Therefore, at the time of removing the absorbent article, it is possible to more reliably separate the stomach-side portion and the back-side portion without breaking the sheet member in a portion excluding the joining portion.

The absorbent article of the present invention may be (3) the absorbent article according to (2), wherein, when the breaking strengths of the portions not including the fused portions in any one of the stomach-side sheet member and the back-side sheet member are regarded as 100%, the breaking strengths in the fused portions are 110% or higher. In the present absorbent article, when the breaking strength of the stomach-side sheet member or the back-side sheet member (not including the fused portions) is regarded as 100%, the lower limit of the breaking strength of the fused portion is 110%. Therefore, it is possible to suppress the circumferential wall portions breaking in a larger number of the fused portions and to make breakage more likely to occur at the boundary portion with the fused portion in the sheet member (not including the fused portion). Therefore, at the time of removing the absorbent article, it is possible to still more reliably facilitate the separation of the stomach-side portion and the back-side portion without breaking the sheet member in a portion excluding the joining portion. It should be noted that there is no particular upper limit of the breaking strength of the fused portion. However, when the breaking strength is too high, the fused portion becomes too stiff, and there is a concern that discomfort may be generated when a wearer or a caregiver comes into contact with the fused portions, and thus the upper limit is preferably 300% or less.

The absorbent article of the present invention may be (4) the absorbent article according to any one of (1) to (3), wherein a dimension of the circumferential wall portion in the thickness direction is larger than a dimension of the core portion in the thickness direction, and a position of the core portion in the thickness direction in each of the plurality of fused portions is shifted toward a side of the stomach-side portion or a side of the back-side portion from a boundary between the stomach-side portion and the back-side portion in the thickness direction. In the present absorbent article, the positions of the core portions are shifted toward the side of the stomach-side portion or the side of the back-side portion from the boundary between the stomach-side portion and the back-side portion in all of the plurality of fused portions in the joining portion. Here, when the joining portion is pulled apart, the fused portion remains in the sheet member where the core portion is present, and a hole is formed due to the removal of the fused portion in the sheet member where the core portion is not present. Therefore, in the present absorbent article, the positions of the core portions are shifted toward a specific side (the side of the stomach-side portion or the side of the back-side portion) in all of the plurality of fused portions in the joining portion, whereby the fused portion is left and the hole is formed on the same side. Therefore, it is possible to more smoothly and appropriately separate the stomach-side portion and the back-side portion at the time of pulling apart the joining portion.

The absorbent article of the present invention may be (5) the absorbent article according to any one of (1) to (4), further including a plurality of elastic members that extend up to the joining portion in the lateral direction and are aligned at gaps in the longitudinal direction in at least one of the stomach-side sheet member and the back-side sheet member, wherein the plurality of elastic members do not overlap the plurality of fused portions in the thickness direction in the joining portion. In the present absorbent article, the plurality of elastic members do not overlap the plurality of fused portions in the thickness direction in the joining portion. Therefore, it is possible to avoid a situation where the elastic members overlap the fused portions, the dimensions (thicknesses) of the circumferential wall portions in the in-plane direction become uneven, and the circumferential wall portion is likely to break. Therefore, at the time of removing the absorbent article, the elastic members disposed in the sheet member have no influence, and it is possible to suppress the circumferential wall portions breaking at a larger number of the fused portions and to make breakage more likely to occur at the boundary portion with the fused portion in the sheet member. In addition, it is possible to suppress a situation where, at the time of removing the absorbent article, the elastic members are separated together with the fused portions and come into contact with the skin of a wearer or caregiver in the middle of removing the absorbent article. Therefore, it is possible to facilitate the more appropriate separation of the joining portion into the stomach-side portion and the back-side portion without breaking the sheet member in a portion excluding the joining portion. It should be noted that the fact that the plurality of elastic members do not overlap the plurality of fused portions means that at least 80% or more of the plurality of elastic members do not overlap the plurality of fused portions.

The absorbent article of the present invention may be (6) the absorbent article according to any one of (1) to (5), wherein the plurality of fused portions include a fused portion in which the core portion has a circular or elliptical shape as seen in the thickness direction. Since the absorbent article has the above-described configuration, in a case where the shape of the core portion is circular or elliptical, the shape drawn by the outer edge of the circumferential wall portion also becomes substantially circular or elliptical. Therefore, at the time of removing the absorbent article, the boundary portion with the fused portion in the sheet member becomes a curved surface with respect to the direction of a force at the time of breaking the boundary portion. Therefore, it is possible to make breakage likely to gradually occur at the boundary portion with the fused portion in the sheet member along the circular or elliptical curved surface in each of the fused portions. Therefore, it becomes possible to facilitate the smooth and appropriate separation of the stomach-side portion and the back-side portion.

The absorbent article of the present invention may be (7) the absorbent article according to any one of (1) to (6), wherein the plurality of fused portions disposed along the longitudinal direction in the joining portion have the same breaking strength. Since the present absorbent article has the above-described configuration, at the time of removing the absorbent article, a wearer or a caregiver is able to continuously and stably pull apart the joining portion along the longitudinal direction all the way to the end using a force of substantially the same magnitude. That is, it becomes possible to facilitate the smooth and appropriate separation of the stomach-side portion and the back-side portion. Here, the expression "the same" mentioned herein means that all of the breaking strengths of the plurality of fused portions are included in a range of the average value of the breaking strengths $\pm$ 10%.

The absorbent article of the present invention may be (8) the absorbent article according to any one of (1) to (7), wherein, in each of the plurality of fused portions, when an area of the core portion is regarded as 100%, an area of the circumferential wall portion is in a range of 5% to 60% as seen in the thickness direction. In the present absorbent article, in each fused portion, when the area of the core portion is regarded as 100%, the lower limit of the area of the circumferential wall portion is 5%. That is, while the main part that plays a function of joining the fused portion is the core portion, the circumferential wall portion can be made stronger by increasing the proportion of the circumferential wall portion in the core portion. Therefore, it is possible to make the fused portion strong (increase the breaking strength) compared with the sheet member and to make breakage likely to occur at the boundary portion with the fused portion in the sheet member. Therefore, at the time of removing the absorbent article, it is possible to further facilitate the separation of the stomach-side portion and the back-side portion without breaking the sheet member in a portion excluding the joining portion. It should be noted that the upper limit of the area of the circumferential wall portion is preferably 60% or less from the viewpoint of the fact that the core portion is the main part that plays a function of joining.

[Advantageous Effect of the Invention]

According to the present invention, in the absorbent article having the joining portion, at the time of removing the absorbent article, it is possible to suppress the breakage of the sheet member in a portion excluding the joining portion and to facilitate the appropriate separation of the stomach-side portion and the back-side portion.

BRIEF DESCRIPTION OF DRAWINGS

Fig. 1 is a perspective view showing a configuration example of a disposable diaper according to an embodiment.
Fig. 2 is a plan view showing the unfolded state of the disposable diaper.
Fig. 3 is a schematic view of a stomach-side portion and a back-side portion in a manufacturing step as seen from the side of end part in the lateral direction.
Fig. 4 is a schematic view for describing a method of forming a joining portion in the manufacturing step.
Fig. 5 is a plan view showing a configuration example of the joining portion in the disposable diaper.

Fig. 6 is a plan view for describing a configuration example of a plurality of fused portions in the joining portion.

Fig. 7 is a plan view and a perspective view showing a configuration example of the fused portion.

Fig. 8 is a schematic view for describing a peel test of the joining portion.

Fig. 9 is a graph showing a relationship between a load and a gap in the peel test.

Fig. 10 is a schematic view for describing the appearance of the fused portion being pulled apart.

Fig. 11 is a plan view for describing another configuration example of the plurality of fused portions in the joining portion.

Fig. 12 is a plan view showing another configuration example of the fused portion.

DESCRIPTION OF EMBODIMENTS

Hereinafter, an absorbent article according to an embodiment of the present invention will be described using a pants-type disposable diaper (hereinafter, also simply referred to as a "disposable diaper") as an example. However, the present invention is not limited to the example and is applicable to a variety of absorbent articles without departing from the subject matter of the present invention. Examples of the absorbent article include so-called three-piece type or two-piece type disposable diapers and disposable diapers in which a joining portion is formed at one of the both (right and left) side parts of a waist opening, but no joining portion is formed at the other side part, and instead, joining (engaging) means (for example, hook-and-loop fasteners) that can be joined (or engaged) to each other is formed.

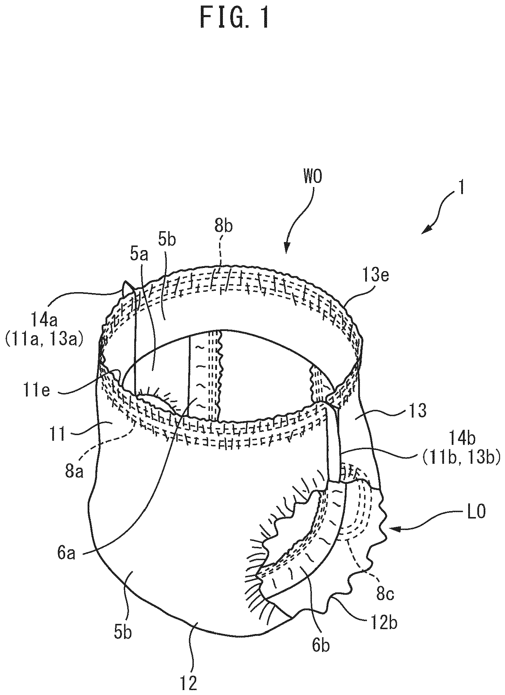

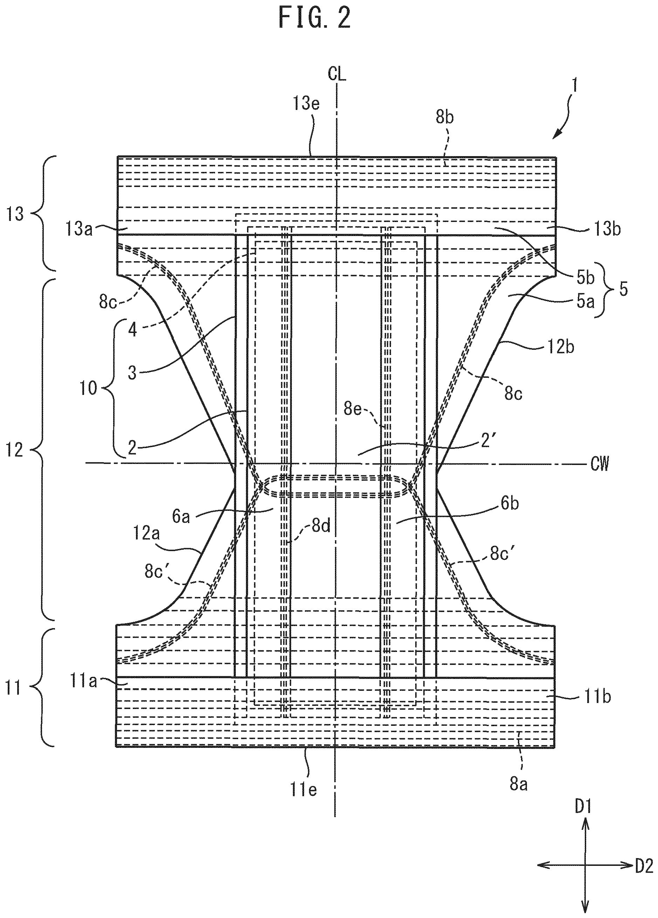

Fig. 1 and Fig. 2 are views showing a disposable diaper 1 according to the present embodiment. Fig.1 is a perspective view showing a configuration example of the disposable diaper 1, and Fig.2 is a plan view showing an unfolded state of the disposable diaper 1. In the state shown in Fig. 2, the disposable diaper 1 has a longitudinal direction L, a lateral direction W, and a thickness direction T that are orthogonal to each other and has a central axis CL that passes through the center in the lateral direction W and extends in the longitudinal direction L and a central axis CW that passes through the center in the longitudinal direction L and extends in the lateral direction W. Here, the orientation toward and the side facing the central axis CL are respectively defined as inward and the inner side in the lateral direction W, and the orientation and the side away from the central axis CL are respectively defined as outward and the outer side in the lateral direction W. The orientation toward and the side facing the central axis CW are respectively defined as inward and the inner side in the longitudinal direction L, and the orientation and the side away from the central axis CW are respectively defined as outward and the outer side in the longitudinal direction L. In addition, the view of the disposable diaper 1 placed on the horizontal plane as seen from vertically above in the thickness direction T is referred to as "plan view", the shape that appears on the plan view is referred to as "planar shape", and a random direction in a plane including the longitudinal direction and the lateral direction W is referred to as "in-plane direction". "Skin side" and "non-skin side" respectively mean sides that are relatively close to and far from the skin surface of a wearer in the thickness direction T of the disposable diaper 1 while the disposable diaper 1 is worn. It should be noted that the respective definitions described above will also be used for each material for the disposable diaper 1 or the disposable diaper 1 in the state shown in Fig. 1.

The disposable diaper 1 includes a stomach-side portion 11, a back-side portion 13, and an intermediate portion 12 between the stomach-side portion 11 and the back-side portion 13. The stomach-side portion 11 is a portion of the disposable diaper 1 that faces the abdomen of a wearer. The intermediate portion 12 is a portion of the disposable diaper 1 that faces the crotch portion of the wearer. The back-side portion 13 is a portion of the disposable diaper 1 that faces the hip portion or back portion of the wearer. The stomach-side portion 11 and the back-side portion 13 are joined to each other with a pair of joining portions 14a and 14b positioned at both ends of the stomach-side portion 11 and the back-side portion 13 in the lateral direction W. The joining portion 14a is formed of one end part 11a of the stomach-side portion 11 in the lateral direction W and one end part 13a of the back-side portion 13 in the lateral direction W that are joined to each other along the longitudinal direction L in a state of overlapping each other in the thickness direction T. Similarly, the joining portion 14b is formed of the other end part 11b of the stomach-side portion 11 in the lateral direction W and the other end part 13b of the back-side portion 13 in the lateral direction W that are joined to each other along the longitudinal direction L in a state of overlapping each other in the thickness direction T. In this case, in the disposable diaper 1, a waist opening WO through which the waist of a wearer passes is defined by an end part 11e of the stomach-side portion 11 on the outer side in the longitudinal direction L and an end part 13e of the back-side portion 13 on the outer side in the longitudinal direction L. In addition, in the disposable diaper 1, a pair of leg openings LO and LO through which the legs of the wearer pass are defined by side parts 12a and 12b of the intermediate portion 12 on both sides. It should be noted that the longitudinal direction, the lateral direction, and the thickness direction of each joining portion 14a or 14b in the state shown in Fig. 1 are the same as the longitudinal direction L, the lateral direction W, and the thickness direction T in the state shown in Fig. 2.

In the present embodiment, in the state shown in Fig. 2, the stomach-side portion 11 and the back-side portion 13 each have an almost rectangular shape that broadens in the lateral direction W and are positioned apart from each other in the longitudinal direction L. The intermediate portion 12 is positioned between the stomach-side portion 11 and the back-side portion 13, and both side edges in the lateral direction W are recessed inward in the lateral direction W. The stomach-side portion 11, the intermediate portion 12, and the back-side portion 13 are integrally formed with each other. In another embodiment (not shown), the stomach-side portion 11, the intermediate portion 12, and the back-side portion 13 are formed separately from each other.

In the present embodiment, the disposable diaper 1 includes a plurality of elastic members 8a or 8b for waist gathers in each of the stomach-side portion 11 and the back-side portion 13. The plurality of elastic members 8a extend along the end part 11e of the stomach-side portion 11 in the longitudinal direction L from one end part 11a to the other end part 11b in the lateral direction W and are disposed at gaps in the longitudinal direction L. Similarly, the plurality of elastic members 8b extend along the end part 13e of the back-side portion 13 in the longitudinal direction L from one end part 13a to the other end part 13b in the lateral direction W and are disposed at gaps in the longitudinal direction L. The plurality of elastic members 8a and 8b stretch and contract the waist opening WO and are exemplified by elastic strings.

In the present embodiment, the disposable diaper 1 includes a plurality of elastic member 8c and 8c' for leg gathers from the intermediate portion 12 to the back-side portion 13 and the stomach-side portion 11. The plurality of elastic members 8c and 8c' are continuously disposed at gaps to extend along the lateral direction W in the center of the intermediate portion 12 in the longitudinal direction L, reach both end parts of the intermediate portion 12 in the lateral direction W, and then extend along the longitudinal direction L up to the end parts 13a and 13b of the back-side portion 13 and the end parts 11a and 11b of the stomach-side portion 11, respectively. The plurality of elastic members 8c and 8c' stretch and contract the pair of leg openings LO and LO respectively and are exemplified by elastic strings.

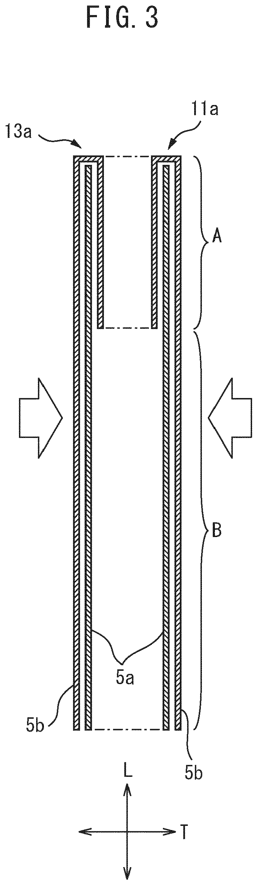

In the present embodiment, the stomach-side portion 11, the intermediate portion 12, and the back-side portion 13 of the disposable diaper 1 are formed of a liquid-impermeable cover sheet (sheet member) 5. Fig. 3 is a schematic view of the stomach-side portion 11 and the back-side portion 13 separately seen from the side of end part in the lateral direction W. Here, the description of the elastic members is omitted. The cover sheet 5 includes a cover sheet 5a that is positioned on the skin side and a cover sheet 5b that is positioned on the non-skin side, and the cover sheet 5a and the cover sheet 5b are laminated together in the thickness direction T and joined to each other with an adhesive or the like. Both end parts of the cover sheet 5b in the longitudinal direction L are folded back toward the skin side to cover both end parts of the cover sheet 5a in the longitudinal direction L. In this case, the cover sheet 5b in the folded-back positions in the stomach-side portion 11 and the back-side portion 13 constitutes the end portion 11e of the stomach-side portion 11 and the end portion 13e of the back-side portion 13, respectively. A region A is a region where three layers of the cover sheet are present, and a region B is a region where two layers of the cover sheet are present. Therefore, the end part 11e of the stomach-side portion 11 and the end part 13e of the back-side portion 13 have a three-layer structure in which three cover sheets 5b, 5a, 5b are laminated together. The length of each three-layer structure in the longitudinal direction L is, for example, 10 mm to 100 mm. In the present embodiment, the plurality of elastic members 8a, 8b, 8c, and 8c' are positioned between the cover sheet 5a and the cover sheet 5b.

Examples of the cover sheet 5 include any of liquid-impermeable sheets such as liquid-impermeable nonwoven fabric or synthetic resin films, composite sheets thereof, SB nonwoven fabric, and SMS nonwoven fabric. Examples of the material of the cover sheet 5 include polyolefin-based materials such as polypropylene or polyethylene. The basis weight of the cover sheet 5 is, for example, 5 to 100 $g/m^2$ and preferably 10 to 50 $g/m^2$. The dimension (thickness) of the cover sheet 5 in the thickness direction T is, for example, 0.2 to 5 mm and preferably 0.2 to 2 mm. In another embodiment, the cover sheet 5 includes one cover sheet or two or more cover sheets (not shown). In still another embodiment, the cover sheet 5b may not be folded back.

In the present embodiment, the disposable diaper 1 includes an absorbent main body 10 on a skin side surface of the cover sheet 5. The absorbent main body 10 includes a liquid-permeable top sheet 2, a liquid-impermeable back sheet 3, and an absorbent body 4 that is positioned between the top sheet 2 and the back sheet 3 and absorbs and holds liquid. Examples of the top sheet 2 include liquid-permeable nonwoven fabric or woven fabric, synthetic resin films having liquid permeable holes formed therein, composite sheet thereof, and the like. Examples of the back sheet 3 include liquid-impermeable nonwoven fabric or synthetic resin films, composite sheets thereof, SMS nonwoven fabric, and the like. In the present embodiment, the absorbent body 4 includes an absorbent body core that absorbs and holds liquid and a core wrap that contains the absorbent body core. As the absorbent body 4, pulp fibers, synthetic fibers, absorbent polymers, or the like is exemplified. The absorbent body 4 is respectively joined to the top sheet 2 and the back sheet 3 with an adhesive or the like, and the top sheet 2 and the back sheet 3 are joined to each other with the adhesive at the circumferential edge parts. The adhesive is a well-known material that is common for the disposable diaper 1, and examples thereof include thermoplastic adhesives.

In the present embodiment, the disposable diaper 1 includes liquid-impermeable leakproof walls 6a and 6b. The leakproof walls 6a and 6b are disposed along the longitudinal direction L on both sides of the top sheet 2 in the lateral direction W. In the end parts of the leakproof walls 6a and 6b on the inner side in the lateral direction W, a plurality of elastic members 8d and 8e that respectively extend in the longitudinal direction L and are aligned at gaps in the lateral direction W are disposed. The plurality of elastic members 8d and 8e respectively stretch and contract the leakproof walls 6a and 6b. As the elastic members 8d and 8e, elastic strings are exemplified.

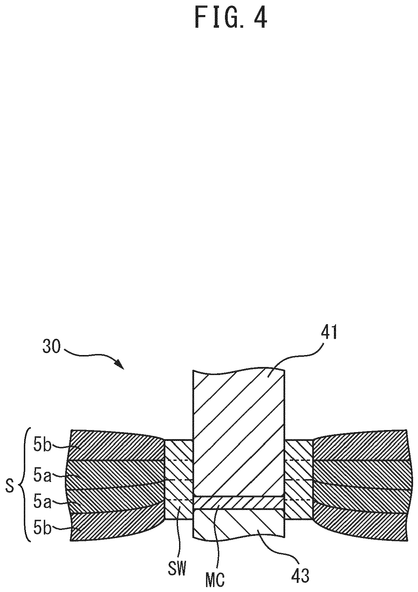

Next, an example of a method of manufacturing the disposable diaper 1 will be described. First, a disposable diaper continuous body (not shown) is formed in which disposable diapers in the unfolded state shown in Fig. 2 are continuously coupled to each other in the lateral direction W. The disposable diaper continuous body has a structure in which the end part 11b of the stomach-side portion 11 and the end part 13b of the back-side portion 13 of one of disposable diapers adjacent to each other are coupled to the end part 11a of the stomach-side portion 11 and the end part 13a of the back-side portion 13 of the other disposable diaper. The disposable diaper continuous body is conveyed in a manner such that the lateral direction W becomes parallel to the machine direction MD. Subsequently, the disposable diaper continuous body is conveyed in the machine direction MD and folded in the longitudinal direction L along the central axis CW with a folding device (not shown). That is, in the disposable diaper continuous body, the end parts 11a of the stomach-side portions 11 and the end parts 13a of the back-side portions 13 of the respective disposable diapers are laminated together, and the end parts 11b of the stomach-side portions 11 and the end parts 13b of the back-side portions 13 are laminated together. Next, in the disposable diaper continuous body, both end parts of the stomach-side portions 11 and the back-side portions 13 are respectively joined to each other with an ultrasonic seal using a joining portion-forming device (not shown). Fig. 4 is a schematic view for describing a method of forming the joining portion. This view shows a part of the region B in FIG. 3. Specifically, the laminated portion of the end part 11a of the stomach-side portion 11 and the end part 13a of the back-side portion 13 and the laminated portion of the end part 11b of the stomach-side portion 11 and the end part 13b of the back-side portion 13 in a disposable diaper continuous body S are each pinched by an ultrasonic horn 43 and a protruding portion 41 of an anvil of the joining portion-forming device. Energy of a predetermined ultrasonic wave is supplied to the pinched portions for a predetermined period of time. Therefore, the plurality of cover sheet 5b, 5a, 5a, and 5b in the laminated portions are fused to each other, thereby forming fused portions 30 each including a core portion MC and a circumferential wall portion SW. Both end parts of the stomach-side portion 11 and the back-side portion 13 are joined to each other with the fused portions 30, and the pair of joining portions 14a and 14b is formed. Examples of the joining portion-forming device include a sealing device described in Patent Literature 2. The shape or the like of the core portion MC or the circumferential wall portion SW can be controlled with the magnitude of the energy to be supplied or the timing or period of the supply of energy. For example, the following control is possible: the conveyance speed is set to be slower than usual, energy is set to be higher than usual, or the size of the protruding portion 41 is set to be smaller than usual, thereby increasing the density per unit area of energy that is to be supplied to a region in which the fused portion of the cover sheets is to be formed and efficiently using energy, which enables energy to be sufficiently given not only to the front end of the protruding portion 41 but also to the circumference of the protruding portion 41. Therefore, it is possible to form not only the core portion MC, which is usually formed at the front end of the protruding portion 41, but also the circumferential wall portion SW in the circumference of the protruding portion 41. It should be noted that the protruding portion 41 may be formed in the ultrasonic horn and the anvil may be formed flat.

Next, the configuration of the joining portions 14a and 14b will be described. Here, the joining portion 14a and the joining portion 14b are almost the same in terms of the configuration except for the difference in position (right and left) in the lateral direction W. Therefore, hereinafter, the configuration of the joining portion 14a will be mainly described, and the configuration of the joining portion 14b will not be described.

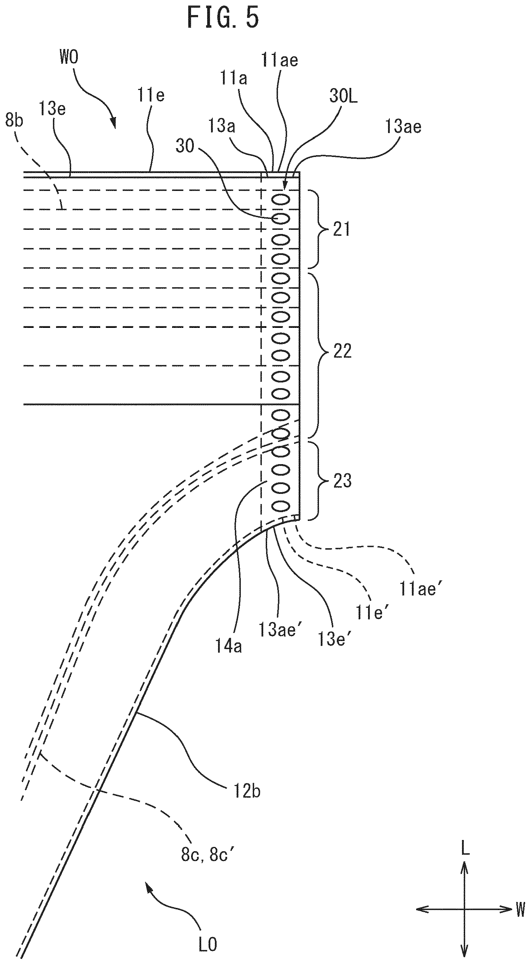

Fig. 5 is a plan view schematically showing a configuration example of the joining portion 14a in the disposable diaper 1. Fig. 5 is a view of the joining portion 14a as seen from the back-side portion 13 side. In the present embodiment, in the joining portion 14a, an end edge 11ae of the end part 11a in the stomach-side portion 11 on the outer side in the longitudinal direction L and an end edge 13ae of the end part 13a in the back-side portion 13 on the outer side in the longitudinal direction L almost overlap each other. The dimensions of the joining portion 14a in the longitudinal direction L and the lateral direction W are, for example, 50 to 250 mm and 3 to 20 mm, respectively. In another embodiment, the end edge 11ae and the end edge 13ae deviate from each other in the longitudinal direction L by a predetermined length (for example, 0.5 to 10 mm) (not shown).

The joining portion 14a includes a plurality of the fused portions 30 that are disposed at gaps along the longitudinal direction L. In this case, the end part 11a and the end part 13a are joined to each other with at least the plurality of fused portions 30. The stomach-side portion and the back-side portion are not joined to each other at portions in the joining portion 14a at which the fused portions 30 are not formed. In another embodiment, portions in the joining portion 14a at which the fused portions 30 are not formed are fully or partially joined to each other with an adhesive (for example, hot-melt adhesive) (not shown).

In the present embodiment, in the joining portion 14a, the plurality of fused portions 30 are aligned side by side at gaps along the longitudinal direction L, thereby constituting a fused portion line 30L. The fused portion line 30L is one line, and the gaps between the fused portions 30 in the longitudinal direction L are constant. However, in another embodiment, a plurality of the fused portion lines 30L are aligned side by side at gaps in the lateral direction W (not shown). In addition, in still another embodiment, in all or part of the fused portion line 30L, the gaps between the fused portions 30 in the longitudinal direction L vary depending on positions (not shown).

In the present embodiment, the plurality of elastic members 8a are provided between the cover sheets 5a and 5b in the stomach-side portion 11 to extend in the lateral direction W up to the joining portions 14a and 14b and to be aligned at gaps in the longitudinal direction L. Similarly, the plurality of elastic members 8b are provided between the cover sheets 5a and 5b in the back-side portion 13 to extend in the lateral direction W up to the joining portions 14a and 14b and to be aligned at gaps in the longitudinal direction L. In addition, the plurality of elastic members 8a and 8b (preferably also the elastic members 8c and 8c') are disposed not to overlap the plurality of fused portions 30 in the thickness directions T at least in the joining portion 14a.

In the present embodiment, the joining portion 14a has, in the longitudinal direction L, a waist-side end part region 21 including the end part on the waist opening WO side, a leg-side end part region 23 including the end part on the leg opening LO side, and a central region 22 between both regions. The waist-side end part region 21 and the leg-side end part region 23 are regions that range from 1/8 to 1/3 of the joining portion 14a in the longitudinal direction L from the end edges of the waist opening WO and the leg opening LO, respectively, and the central region 22 is the remaining region. The waist-side end part region 21 and the leg-side end part region 23 have the same length in the longitudinal direction L. In each region, the fused portions 30 are disposed in the same number of lines at the same gap (with a manufacturing error). In another embodiment, the waist-side end part region 21 and the leg-side end part region 23 have different lengths in the longitudinal direction L (not shown). In another embodiment, in at least one region, at least one of the size, gaps, and number of lines of the fused portions 30 is different from that of other regions (not shown).

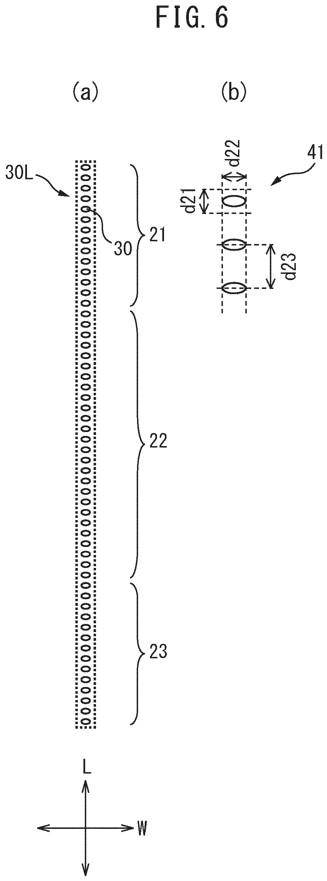

Fig. 6 is a plan view for describing a configuration example of the plurality of fused portions 30 in the joining portion 14a. Fig. 6(a) shows the fused portions 30 (fused portion line 30L) as seen from the side of the back-side portion 13 in the thickness direction T, and Fig. 6(b) shows the shape and disposition of the protruding portions 41 at the front end part in the anvil in which the fused portions 30 are formed by an ultrasonic sealing method. In the present embodiment, as the fused portion line 30L, the plurality of fused portions 30 in the waist-side end part region 21, the central region 22, and the leg-side end part region 23 all have the same planar shape. In other words, in the case of forming the respective fused portions 30 in the respective regions by the ultrasonic sealing method, the planar shapes of the protruding portions 41 of the anvil for the respective fused portions 30 in the respective regions are the same. Here, the planar shape of the protruding portion 41 is not particularly limited, and examples thereof include a circular shape, an elliptical shape, a rectangular shape, a rounded rectangular shape, a polygonal shape, and the like. In the present embodiment, the planar shape of the protruding portion 41 is an elliptical shape, a dimension d21 (minor axis) in the longitudinal direction L is smaller than a dimension d22 (major axis) in the lateral direction W, and the sizes thereof are, for example, 0.5 mm to 5 mm. A distance d23 between the protruding portions 41 adjacent to each other in the longitudinal direction L (the distance between the major axes) is, for example, 1 mm to 30 mm.

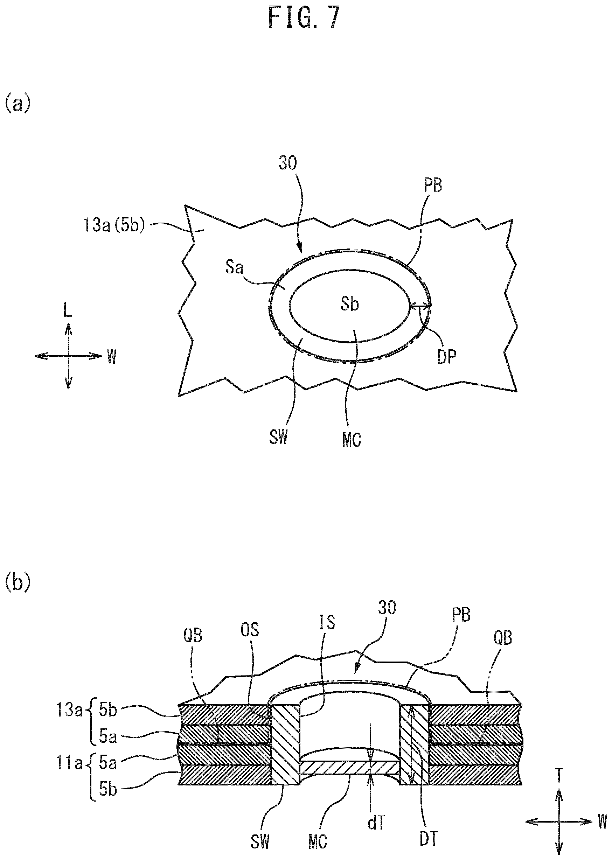

Next, the configuration of the fused portion 30 will be further described. Here, the configuration of the fused portion 30 in the central region 22 will be described. It should be noted that the configuration of the fused portion 30 in the leg-side end part region 23 and the configuration of the fused portion 30 in the waist-side end part region 21 (excluding the number of the cover sheets 5) are also the same as the configuration of the fused portion 30 in the central region 22 and thus will not be described again. Fig. 7 is a plan view and a perspective view showing an example of the configuration of the fused portion 30. Here, Fig. 7(a) is a plan view of the fused portion 30 in the central region 22, and Fig. 7(b) is a perspective view including a cross section of the fused portion 30 in the central region 22 as seen in the longitudinal direction L. The fused portion 30 includes the core portion MC and the circumferential wall portion SW.

In the present embodiment, the core portion MC has a configuration in which the plurality of cover sheets 5a and 5b in the stomach-side portion 11 and the plurality of cover sheets 5a and 5b in the back-side portion 13 are fused to each other in the thickness direction T. The core portion MC is formed of the plurality of cover sheets 5b, 5a, 5a, and 5b pinched between the front end part of the ultrasonic horn and the protruding portion of the anvil that are melted by energy that is supplied from the ultrasonic horn, compressed in the thickness direction T, and solidified. Therefore, the core portion MC is joined extremely strongly in the thickness direction T. A dimension (thickness) dT of the core portion MC in the thickness direction T is, for example, 20 to 500 $\mu$m. The dimension dT of the core portion MC is significantly smaller than the dimension of the plurality of cover sheets 5b, 5a, 5a, and 5b in the stomach-side portion 11 and the back-side portion 13 that are simply laminated together in the thickness direction T (for example, 200 to 5000 $\mu$m). When dT is smaller than 20 $\mu$m, the cover sheets are not stably fused to each other, and a hole is likely to be opened during fusion. When dT exceeds 500 $\mu$m, the cover sheets are not fully fused to each other, and there is a concern that the cover sheets are likely to break.

In addition, in the present embodiment, the planar shape of the core portion MC is substantially the same as the planar shape of the protruding portion 41 of the anvil, and examples thereof include a circular shape, an elliptical shape, a rectangular shape, a rounded rectangular shape, a polygonal shape, and the like. In the present embodiment, the planar shape of the core portion is an elliptical shape. At this time, the dimension in the longitudinal direction L and the dimension in the lateral direction W are substantially the same as the dimension d21 in the longitudinal direction L and the dimension d22 in the lateral direction W of the protruding portion 41 and are 0.5 mm to 5 mm. In a case where each dimension of the core portion MC is smaller than 0.5 mm, the fused portions 30 are too small and likely to break, and there is a concern that the joining portion 14a may unintentionally peel off while the disposable diaper 1 is worn. In a case where each dimension of the core portion MC exceeds 5 mm, the fused portion 30 is too large, and there is a concern that a wearer may sense discomfort by feeling hardness. In addition, the distance between the core portions MC adjacent to each other is substantially the same as the distance between the protruding portions 41 adjacent to each other and is 1 mm to 30 mm. In a case where the distance is smaller than 1 mm, the number of the fused portions 30 in the joining portion 14a increases, and there is a concern that the disposable diaper 1 may not be easily pulled apart when attempted to be removed. In a case where the distance exceeds 30 mm, the number of the fused portions 30 decreases, and there is a concern that the joining portion 14a may unintentionally peel off while the disposable diaper 1 is worn.

Furthermore, in the present embodiment, in the fused portion 30, the position of the core portion MC in the thickness direction T is shifted toward the stomach-side portion 11 or the back-side portion 13 from the boundary between the stomach-side portion 11 and the back-side portion 13. Here, the position of the core portion MC in the thickness direction T is defined as the position of the center of the core portion MC in the thickness direction T. In a case where the core portion MC is not flat, the position of the core portion MC in the thickness direction T is defined as the position of the center of the core portion MC in the thickness direction T in the coupling portion of the core portion MC and the circumferential wall portion SW in the longitudinal direction L. For example, in the example of Fig. 7(b), the position of the core portion MC in the thickness direction T is shifted toward the stomach-side portion 11 side and is a position between the cover sheet 5a and the cover sheet 5b of the stomach-side portion 11. The shift in the thickness direction T from the boundary between the stomach-side portion 11 and the back-side portion 13 is, for example, 10 to 500 $\mu$m. In another embodiment, in the thickness direction T, the position of the end surface of the core portion MC coincides with the position of the end surface of the cover sheet 5 in the stomach-side portion 11 or the back-side portion 13 (not shown).

In the present embodiment, the circumferential wall portion SW has a configuration in which the plurality of cover sheets 5a, 5b of the stomach-side portion 11 and the plurality of cover sheets 5a, 5b of the back-side portion 13 are fused to each other in the thickness direction T to extend in the thickness direction T from the circumferential edge of the core portion MC while surrounding the core portion MC in a tubular shape. In the present embodiment, the circumferential wall portion SW is formed of the plurality of cover sheet 5b, 5a, 5a, and 5b that are melted by energy that is supplied from the front end part of the ultrasonic horn or heat generated during the fusion of the core portion MC, dragged due to the compression of the core portion MC, slightly compressed in the thickness direction T, and solidified. Therefore, the circumferential wall portion SW is joined relatively strongly in the thickness direction T. A dimension (thickness) DT of the circumferential wall portion SW in the thickness direction T is, for example, 100 to 1000 $\mu$m. The dimension DT of the circumferential wall portion SW is smaller than the dimension of the plurality of cover sheets 5b, 5a, 5a, and 5b in the stomach-side portion 11 and the back-side portion 13 that are simply laminated together in the thickness direction T (for example, 200 to 5000 $\mu$m). However, since the circumferential wall portion SW is not pressurized by the front end part of the ultrasonic horn or the protruding portion 41 of the anvil, the dimension DT of the circumferential wall portion SW is larger than the dimension dT of the core portion MC (for example, 20 to 500 $\mu$m). When the size of DT is smaller than 100 $\mu$m, the breaking strength of the circumferential wall portion SW becomes weak, and there is a concern that the cover sheet may break in an unexpected direction at the time of removing the disposable diaper. When the size of DT exceeds 1000 $\mu$m, there is a concern that a wearer or a caregiver may sense discomfort when the circumferential wall portion comes into contact with the skin of the wearer or the caregiver.

Here, the circumferential wall portion SW has a tubular form and has an outer surface OS that constitutes the surface of the tube on the outer side and an inner surface IS that constitutes the surface of the tube on the inner side. Therefore, the dimension of the circumferential wall portion SW in the in-plane direction (thickness) can be said as the distance between the outer surface OS and the inner surface IS. When the fused portion 30 is cut in a cross section parallel to the thickness direction T, the outer surface OS and the inner surface IS are the surface on the outer side and the surface on the inner side of the circumferential wall portion provided to stand along the thickness direction T from the circumferential edge of the core portion MC in the cross section. That perpendicular cross section can be obtained by observation with, for example, a scanning electron microscope. The outer surface OS is a boundary surface between a portion of the cover sheet 5 where there is no fused portion 30 and the fused portion 30. Therefore, the outer surface OS is a surface from which fibers in the cover sheet 5 are released, and accordingly, the outer surface OS is a somewhat uneven surface. On the other hand, the inner surface IS is a surface in contact with the protruding portion 41 of the anvil. Therefore, although there is a case where the fibers in the cover sheet 5 remain in a part of the inner surface IS, the inner surface IS is a relatively flat surface. In addition, the outer surface OS and the inner surface IS do not necessarily intersect perpendicularly with the surface of the core portion MC and may be inclined to some extent (for example, $\pm 30°$ with respect to the vertical direction). Here, the tubular form (or tubular shape) refers to a long and narrow hollow shape or a pipe shape. The tubular form may bend or meander as long as the tubular shape extends in the thickness direction T, may have a variety of shapes such as a circular shape, an elliptical shape, a polygonal shape, and a combined shape on a cross section perpendicular to the thickness direction T, and may have a shape and an area on a cross section perpendicular to the thickness direction T that are not constant in the thickness direction T.

In addition, in the present embodiment, the planar shape of the circumferential wall portion SW has a ring shape that surrounds the core portion MC in a substantially even width DP, and examples thereof include a circular ring, an elliptical ring, a rectangular ring, a rounded rectangular ring, a polygonal ring, and the like. In the present embodiment, the planar shape of the circumferential wall portion SW is an elliptical ring. In addition, in the circumferential wall portion SW, the variation of the width DP depending on the position in the ring is small, the width DP rarely becomes partially thin or thick abruptly, and the shape is not uneven. That is, the width DP (or wall thickness) of the circumferential wall portion SW in the in-plane direction is not uneven. Here, the expression "uneven" refers to the fact that, in the circumferential wall portion SW, there is a place where the width DP becomes as small a value as equal to or smaller than 1/2 of the average value or a place where the width DP becomes as large a value as equal to or larger than twice the average value. It should be noted that, when the width DP is not constant in the thickness direction T, the width at the boundary between the stomach-side portion 11 and the back-side portion 13 is used. The size of the width DP is, for example, 50 to 400 μm. In a case where the width DP is smaller than 50 μm, the circumferential wall portion SW is too thin and is likely to break, and there is a concern that the joining portion 14a may unintentionally peel off while the disposable diaper 1 is worn. In a case where DP exceeds 400 μm, the circumferential wall portion SW is too thick, and there is a concern that a wearer may sense discomfort by feeling hardness.

In the present embodiment, in the fused portion 30, an area Sa of the circumferential wall portion SW is in a range of 5% to 60% as seen in the thickness direction T (in a plan view) when an area Sb of the core portion MC is regarded as 100%. The area Sa of the circumferential wall portion SW is preferably in a range of 5% to 55% and more preferably in a range of 5% to 50% of the area Sb of the core portion MC. When the area Sa of the circumferential wall portion SW is less than 5%, the strength of the circumferential wall portion SW decreases, and there is a concern that the circumferential wall portion SW itself may break at the time of removing the disposable diaper. When the area Sa of the circumferential wall portion SW exceeds 60%, the circumferential wall portion SW becomes too large, and there is a concern that a wearer may sense discomfort by feeling hardness.

The evenness of the width DP of the circumferential wall portion SW can be evaluated by, for example, a peel test between the cover sheet 5 in the stomach-side portion 11 and the cover sheet 5 in the back-side portion 13 in the joining portion 14a of the disposable diaper 1. Hereinafter, the evaluation method will be specifically described.

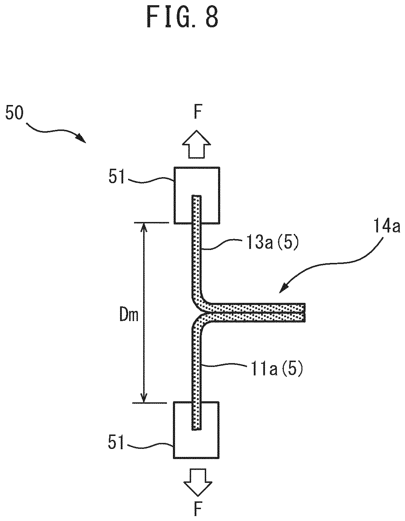

Fig. 8 is a schematic view for describing the peel test of the joining portion 14a. In the present embodiment, the peel test of the joining portion is a T-type peel test and is a test where a portion including at least a part of the joining portion 14a is used as a sample, the end parts on the same side of the cover sheets 5 and 5 in the stomach-side portion 11 and the back-side portion 13 in the sample are respectively gripped by two chucks 51 and 51 provided in a tester for the peel test, the two chucks 51 and 51 are pulled away at a constant speed to broaden a gap Dm between the two chucks 51 and 51 and thereby peel the joining portion 14a, and the peel strength of the joining portion 14a is measured based on the relationship between a load that is applied between the two chucks 51 and 51 and the gap between the two chucks 51 and 51. Specifically, the T-type peel test is performed as described below.

<A. T-type peel test of joining portion>

(1) A portion including a part of the joining portion 14a (including the cover sheet 5 in the stomach-side portion 11 (stomach-side sheet member) and the cover sheet 5 in the back-side portion 13 (back-side sheet member)) in the disposable diaper 1 is cut out to a size of 25 mm in the longitudinal direction L and 50 mm in the lateral direction W and used as a sample.

(2) A tester 50 for the peel test is used, the end parts of the cover sheet 5 in the stomach-side portion 11 and the cover sheet 5 in the back-side portion 13 of the cut sample on the same side in the lateral direction W (the central side of the disposable diaper 1 before being cut out) are respectively gripped by the two chucks (jigs) 51 and 51 provided in the tester 50. Here, the distance (initial value) between the chucks at the time of gripping the end parts is set to 30 mm in advance.

(3) In the tester 50, the two chucks 51 and 51 are pulled away at a constant speed (for example: 100 mm/min) such that the gap Dm between the two chucks 51 and 51 broadens, thereby measuring the gaps Dm between the two chucks 51 and 51 and a load F that is applied to the two chucks 51 and 51 while peeling the cover sheet 5 in the stomach-side portion 11 and the cover sheet 5 in the back-side portion 13 in the joining portion 14a in the 180° direction.

(4) Based on the relationship between the load F that is applied between the two chucks 51 and 51 and the gap Dm between the two chucks 51 and 51, the peel strength of the joining portion 14a is measured. The maximum value of the measured loads F is regarded as the peel strength (N/25 mm). Therefore, the peel strength for joining with a specific number (m) of the fused portions 30 (for example, m = 6) included in the portion of the joining portion 14a that is 25 mm long in the longitudinal direction L is measured. In this case, the result from a single measurement can be regarded as, for example, the total value or the averaged value of the specific number (m) of the fused portions 30. Here, the measurement through (1) to (4) is performed such that all (for example, 60) of the plurality of fused portions 30 included in the joining portion 14a are evaluated. For example, in a case where the total number of the fused portions 30 is 60 and six fused portions 30 (m=6) are measured each measurement, at least 10 measurements are performed.

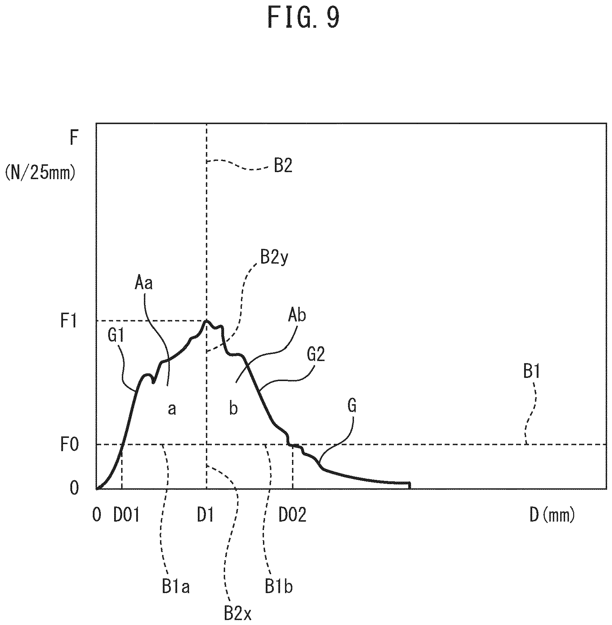

Fig. 9 is a graph G showing an example of the relationship between the load F and the gap D in the peel test of FIG. 8. Here, the horizontal axis indicates the gap D (= Dm - 30 (initial value)) (mm) between the two chucks 51 and 51, and the vertical axis indicates the load F (N/25 mm) that is applied between the two chucks 51 and 51. Here, in the region indicated by the graph G showing the relationship between the gap D and the load F, a specific region where the load F is equal to or greater than a predetermined reference load F0, that is, the gap D is D01 to D02 will be considered. The specific region is divided into, with respect to a maximum load gap D1, which is the gap D where the load F shows the maximum value F1, as the boundary, a short-gap side region Aa on the short-gap side and a long-gap side region Ab on the long-gap side. In Fig. 9, the short-gap side region Aa is a region that is surrounded by a dashed line B1a, which is a dashed line B 1 indicating the reference load F0 along the gap D in a range of D01 to D1, a solid line G1, which is a solid line in the graph G along the gap D in a range of D01 to D1, and a dashed line B2y, which is a dashed line B2 indicating the maximum load gap D1 along the load F in a range of F0 to F1. Similarly, in Fig. 9, the long-gap side region Ab is a region that is surrounded by a dashed line B1b, which is a dashed line B 1 indicating the reference load F0 along the gap D in a range of D1 to D02, a solid line G2, which is a solid line in the graph G along the gap D in a range of D1 to D02, and the dashed line B2y, which is the dashed line B2 indicating the maximum load gap D1 along the load F in a range of F0 to F1.

It should be noted that the predetermined reference load is a load necessary to pull apart the cover sheet 5 in the stomach-side portion 11 and the cover sheet 5 in the back-side portion 13 in a portion excluding the fused portion 30 in the sample and is a load relating to the peeling of joining in a portion excluding the fused portion 30 (for example, natural entanglement between fibers, joining with an adhesive, or the like). In this sample (25 mm in the longitudinal direction L and 50 mm in the lateral direction W), such a load is, for example, 4 N/25 mm.

At this time, in the present embodiment, the ratio a/b between the area a of the short-gap side region Aa and the area b of the long-gap side region Ab is 0.5 or more and 2.0 or less. Therefore, it can be said that a portion where the width DP (wall thickness) of the circumferential wall portion SW is uneven is small or that the evenness of the width DP is high. Here, from the viewpoint of further enhancing the evenness of the width DP, the ratio a/b is preferably 0.7 or more and 1.8 or less and more preferably 0.8 or more and 1.5 or less.

Here, the reason for the fact that the width DP of the circumferential wall portion SW is not uneven and the evenness of the width DP is relatively high when the ratio a/b is 0.5 or more and 2.0 or less is considered as described below.

In the graph G showing the peel strength of the joining portion 14a, the portion with the load that is equal to or greater than the predetermined reference load relates to the strength for peeling joining with the fused portion 30 (not with an adhesive or the like). That is, in the graph G, the portion with the load that is equal to or greater than the reference load can be said to indicate the load relating to the breakage of (the circumferential wall portion SW of) the fused portion 30 and the cover sheet 5 in the circumference of the fused portion 30. Therefore, the short-gap side region Aa indicates the breakage of half of the fused portions 30 on the front side in the peeling direction of the sample with the tester 50 and the cover sheet 5 in the circumference of the fused portions 30 (hereinafter, also referred to as "front half portion"). On the other hand, the long-gap side region Ab indicates the breakage of half of the fused portions 30 on the back side in the peeling direction of the sample and the cover sheet 5 in the circumference of the fused portions 30 (hereinafter, also referred to as "back half portion"). Therefore, in the specific region indicated by the graph G of the peel strength described above, the fact that the area a of the short-gap side region Aa and the area b of the long-gap side region Ab are substantially the same as each other means that the energy for the breakage of the front half portion and the energy for the breakage of the back half portion are substantially the same as each other. In this case, the breakage of the front half portion and the breakage of the back half portion are considered to proceed in substantially the same manner. That is, the state of breakage between the half of the fused portions on the front side and the sheet member in the circumference of the above-described fused portions and the state of breakage between the half of the fused portions on the back side and the sheet member in the circumference of the above-described fused portions are considered to be substantially the same as each other. Therefore, it can be said that, in the circumferential wall portion SW, a place where the width becomes partially too small is not generated. In other words, it can be said that, in the circumferential wall portion SW, there are only a small portion where the dimension in the in-plane direction, that is, the width DP (wall thickness) is uneven. Here, in consideration of the characteristics of products or manufacturing, it is difficult to make the area a of the short-gap side region Aa and the area b of the long-gap side region Ab strictly the same as each other, that is, to achieve the ratio a/b = 1. However, in order to achieve a state where there are only a small portion where the width DP (wall thickness) of the circumferential wall portion SW is uneven, the area a of the short-gap side region Aa and the area b of the long-gap side region Ab need to be substantially the same as each other, and thus, specifically, it is conceivable that the ratio a/b may be 0.5 or more and 2.0 or less.

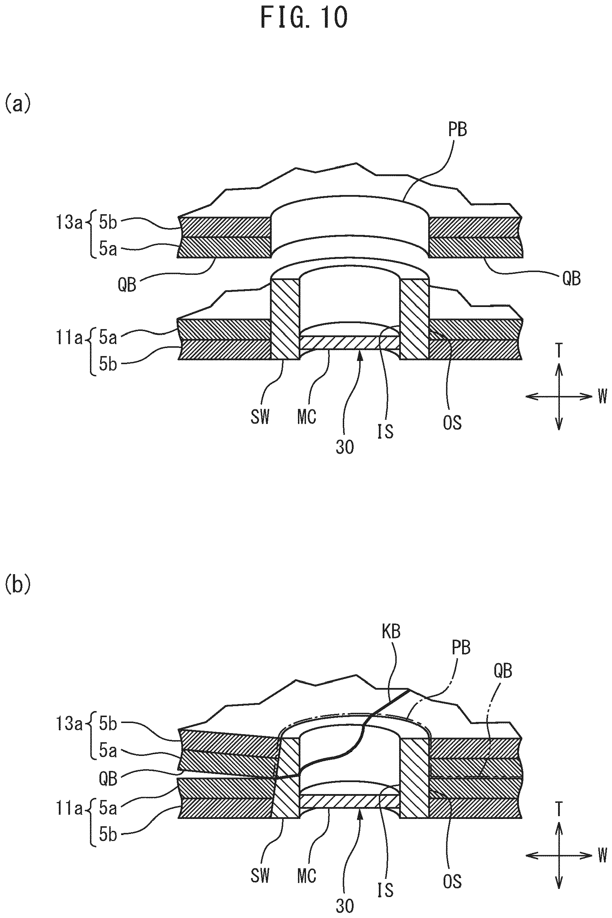

The action and effect of the joining portion 14a having the above-described configuration will be described with reference to Fig. 7 and Fig. 10. Fig. 10 is a schematic view for describing the appearance of the fused portion 30 being pulled apart. In the disposable diaper 1 of the present embodiment, each fused portion 30 in the joining portion 14a has the core portion MC and the circumferential wall portion SW. Here, the circumferential wall portion SW is formed of the cover sheet 5 in the stomach-side portion 11 (stomach-side sheet member) and the cover sheet 5 in the back-side portion 13 (back-side sheet member) that are melted, integrated in the thickness direction, and solidified in a tubular wall shape. Therefore, usually, the circumferential wall portion SW is strong and does not easily break compared with the cover sheet 5 in the stomach-side portion 11 or the back-side portion 13 in the circumference of the circumferential wall portion SW. Therefore, in the case of peeling the joining portion 14a, the circumferential wall portion SW itself is less likely to break, and the cover sheet 5 in the stomach-side portion 11 or the back-side portion 13 in the circumference of the circumferential wall portion SW is likely to break at a boundary portion PB with the circumferential wall portion SW or the circumferential portion of the boundary portion PB (hereinafter, simply referred to as "boundary portion PB"). Therefore, as shown in Fig. 7 (b), when the joining portion 14a is pulled apart to remove the disposable diaper 1, first, in a portion excluding the fused portion 30, it is possible to easily pull apart the joining portion 14a at a boundary QB between the cover sheet 5 in the stomach-side portion 11 and the cover sheet 5 in the back-side portion 13. In addition, in the fused portion 30 and the circumference of the fused portion 30, breakage is made likely to occur at the boundary portion PB with (the circumferential wall portion SW of) the fused portion 30 in the cover sheet 5 in the stomach-side portion 11 or the back-side portion 13. As a result, it is possible to make it easy, for example, to leave the fused portion 30 in the cover sheet 5 in the stomach-side portion 11 and to form a hole due to the removal of the fused portion 30 in the cover sheet 5 in the back-side portion 13 as shown in Fig. 10(a). Therefore, it is possible to continuously and easily pull apart the portion of the stomach-side portion 11 and the portion of the back-side portion 13 in the joining portion 14a from each other in the longitudinal direction L.

However, in a case where the width DP of the circumferential wall portion SW is uneven, for example, in a case where the width DP becomes partially small, the circumferential wall portion SW of the fused portion 30 becomes partially not strong. Therefore, breakage or cracking is likely to occur not in the fused portion 30 in the cover sheet 5 in the stomach-side portion 11 or the back-side portion 13, that is, the boundary portion PB with the circumferential wall portion SW, but in the circumferential wall portion SW itself (a portion with a small width DP). In a case where the number of such fused portions 30 (circumferential wall portions SW) is relatively small, there is a low possibility that breakage or cracking may actually occur in the circumferential wall portion SW itself. However, in a case where the number is relatively large, there is a high possibility that breakage or cracking may actually occur in the circumferential wall portion SW itself. In such a case, there is a possibility that, as shown in Fig. 10(b), for example, in the middle of pulling apart the joining portion 14a, a crack KB generated in the circumferential wall portion SW of the fused portion 30 may proceed to the cover sheet 5 in a portion excluding the joining portion 14a in the stomach-side portion 11 or the back-side portion 13, for example, the cover sheet 5 in the central portion of the stomach-side portion 11 or the back-side portion 13. As a result, there is a concern that it may become difficult to continuously pull apart the portion of the stomach-side portion 11 and the portion of the back-side portion 13 in the joining portion 14a from each other in the longitudinal direction L.

However, in the disposable diaper 1 of the present embodiment, an effort is made in a manner such that a/b, which is the ratio of the area a of the short-gap side region Aa and the area b of the long-gap side region Ab, becomes 0.5 or more and 2.0 or less as described above. That is, an effort is made in a manner such that the area a of the short-gap side region Aa and the area b of the long-gap side region Ab become substantially the same as each other. Therefore, the energy for the breakage of the front half portion and the energy for the breakage of the back half portion in the fused portions 30 and the cover sheet 5 in the circumference of the fused portions 30 are substantially the same as each other. Therefore, the breakage of the front half portion and the breakage of the back half portion are considered to proceed in substantially the same manner. That is, the state of breakage between the half of the fused portions 30 on the front side and the cover sheet 5 in the circumference of the fused portions 30 and the state of breakage between the half of the fused portions 30 on the back side and the cover sheet 5 in the circumference of the fused portions 30 are considered to be substantially the same as each other. Therefore, it can be said that, in the circumferential wall portion SW, a place where the width becomes partially too small is not generated. In other words, it can be said that, in the circumferential wall portion SW, there are only a small number of places where the width is uneven.

As described above, in the disposable diaper 1 of the present embodiment, the width of the circumferential wall portion SW is relatively even as a whole in the fused portion 30. Therefore, when the stomach-side portion 11 and the back-side portion 13 are pulled apart from each other as shown in Fig. 10(a), it is possible to suppress the circumferential wall portion SW itself being broken due to a force exerted to pull apart the stomach-side portion 11 and the back-side portion 13, and it is possible to make breakage likely to occur at the boundary portion PB with the fused portion 30 (circumferential wall portion SW) in the cover sheet 5 in the stomach-side portion 11 or the back-side portion 13. That is, it is possible to relatively decrease the number of fused portions 30 that break in the circumferential wall portion SW itself. As a result, it is possible to easily pull apart the joining portion 14a into the stomach-side portion 11 and the back-side portion 13 along the longitudinal direction L all the way to the end, and it is possible to suppress a phenomenon in which the circumferential wall portion SW breaks, the breakage proceeds from the broken portion, and the cover sheet 5 in a portion excluding the joining portion 14a breaks. As described above, in the disposable diaper 1 having the joining portion 14, it is possible to facilitate, at the time of removing the disposable diaper 1, the appropriate separation of the joining portion 14 into the stomach-side portion 11 and the back-side portion 13 without breaking the cover sheet 5 in a portion excluding the joining portion 14. It should be noted that, when the joining portion 14a is pulled apart into the stomach-side portion 11 and the back-side portion 13 all the way to the end, among the plurality of fused portions 30 in the joining portion 14a, as many fused portions 30 as possible preferably satisfy the numerical range of the a/b. In such a case, it is possible to suppress the breakage of the sheet member in a portion excluding the joining portion 14a and to facilitate the appropriate pulling-apart of the joining portion 14a into the stomach-side portion 11 and the back-side portion 13. As many fused portions 30 as possible refer to 90% or more, preferably 95% or more, and more preferably 100% of the fused portions 30. For example, in the case of being recognized by the T-type peel test described above, as many fused portions 30 as possible can be said as described below. For example, in a case where the total number of the fused portions 30 is 60, and six (m = 6) fused portions 30 are measured in a single measurement, out of 10 measurements, the numerical range of the a/b is preferably satisfied in at least 9 measurements (90%) and more preferably satisfied in 10 measurements (100%). Alternatively, for example, in the T-type peel test described above, in a case where the total number of the fused portions 30 is 60, and three (m = 3) fused portions 30 are measured in a single measurement, out of 20 measurements, the numerical range of the a/b is preferably satisfied in at least 18 measurements (90%), more preferably satisfied in at least 19 measurements (95%), and still more preferably satisfied in 20 measurements (100%). In addition, particularly, in the case of assuming that the joining portion 14 is pulled apart from the waist opening WO side, 90% or more of the fused portions 30 that are continuous from the waist opening WO side preferably satisfy the numerical range. On the other hand, in the case of assuming that the joining portion 14 is pulled apart from the leg opening LO side, 90% or more of the fused portions 30 that are continuous from the leg opening LO side preferably satisfy the numerical range. In such a case, at the time of removing the disposable diaper 1, it is possible to further suppress the breakage of the sheet member in a portion excluding the joining portion 14a so that appropriate separation of the stomach-side portion and the back-side portion can be more easily achieved.

In the present embodiment, the breaking strength in the fused portion 30 is higher than the breaking strength of a portion not including the fused portion 30 in any one of the cover sheet 5 in the stomach-side portion 11 (stomach-side sheet member) and the cover sheet 5 in the back-side portion 13 (back-side sheet member) that constitute the joining portion 14a.

As described above, the disposable diaper 1 has the above-described breaking strength relationship, whereby, when the stomach-side portion 11 and the back-side portion 13 are pulled apart from each other at the time of removing the disposable diaper 1, it is possible to make breakage more likely to occur at the boundary portion PB with the fused portion 30 in the cover sheet 5 (not including the fused portion). Therefore, the breakage of the circumferential wall portions SW in a larger number of the fused portions 30 is suppressed, whereby it is possible to make it easy, for example, to leave the fused portion 30 in the cover sheet 5 in the stomach-side portion 11 and to form a hole due to the removal of the fused portion 30 in the cover sheet 5 in the back-side portion 13. Therefore, it is possible to continuously and easily pull apart the portion of the stomach-side portion 11 and the portion of the back-side portion 13 in the joining portion 14a from each other in the longitudinal direction L. That is, at the time of removing the disposable diaper 1, it is possible to more reliably separate the stomach-side portion 11 and the back-side portion 13 without breaking the cover sheet 5 in a portion excluding the joining portion 14a.

Here, the breaking strength of the fused portion 30 and the breaking strengths of the cover sheets 5 (not including the fused portion 30) of the stomach-side portion 11 and the back-side portion 13 are respectively measured as described below.

<B. Breaking strength of fused portion>

(1) Since the size of the fused portion 30 is too small, and it is difficult to measure the breaking strength of the fused portion 30 itself, for convenience, a sample that simulates the fused portion 30 in a pseudo manner is prepared by the following method.

(1-1) The material for the cover sheet 5 in the stomach-side portion 11 and the material for the cover sheet 5 in the back-side portion 13 are overlapped together. Next, the overlapped materials are joined together by heat-sealing while being pressurized using a sealing machine (manufactured by Fujiimpulse Co., Ltd.: POLY SEALER, P-300). The heating time is one second, the heated length is 200 mm, and the heated width is 5mm. Therefore, a laminate of the materials is formed. In the laminate, for example, a fused portion having a length of 200 mm and a width of 5 mm is formed. Here, the 5 mm width is matched to the dimension (width) of the fused portion 30 in the joining portion 14 in the lateral direction W (error $\pm$ 30%).

(1-2) The laminate is cut out to a size of 150 mm $\times$ 5 mm and used as a sample. At this time, in the sample, a core portion having a length of 150 mm and a width of 2 mm is formed.

(2) A tensile tester including a load cell having a maximum load capacity of 50 N (manufactured by Shimadzu Corporation: AUTOGRAPH, AGS-lkNG) is used, and both end parts of the sample in a second direction are respectively gripped with two chucks provided in the tensile tester. Here, the distance (initial value) between the chucks at the time of gripping the end parts is set to 100 mm in advance.

(3) In the tensile tester, the load that is applied to the two chucks is measured while pulling apart the two chucks at a constant speed (for example: 100 mm/min) such that the gap between the two chucks broadens.

(4) The load at the time of the breakage of the sample is regarded as the breaking strength. Here, the average value of the breaking strengths of six samples is calculated as the final breaking strength (N/5mm). It should be noted that, although the fused portion of the sample is different from the fused portion 30 of the joining portion 14, the breaking strength can be substantially matched by substantially matching the width of the fused portion in the sample to the dimension (width) of the fused portion 30 in the lateral direction W.

<C. Breaking strengths of cover sheets in stomach-side portion and back-side portion>

(1) The material for the cover sheet 5 in the stomach-side portion 11 or the back-side portion 13 is cut out to a size of 150 mm $\times$ 5 mm (longitudinal direction $\times$ width direction) and used as a sample.

(2) A tensile tester including a load cell having a maximum load capacity of 50 N (manufactured by Shimadzu Corporation: AUTOGRAPH, AGS-1kNG) is used, and both end parts of the sample in the longitudinal direction are respectively gripped with two chucks provided in the tensile tester. Here, the distance (initial value) between the chucks at the time of gripping the end parts is set to 100 mm in advance.

(3) In the tensile tester, the load that is applied to the two chucks is measured while pulling apart the two chucks at a constant speed (for example: 100 mm/min) such that the gap between the two chucks broadens.

(4) The load at the time of the breakage of the sample is regarded as the breaking strength. Here, the average value of the breaking strengths of six samples is calculated as the final breaking strength (N/5mm).

Here, in the B, for example, the breaking strength of the sample, which is prepared by laminating four sheets of the material for the cover sheet 5, corresponds to that of the joining portion 14a in Fig. 7, in which the (two) cover sheets 5b and 5a in the stomach-side portion 11 and the (two) cover sheet 5a and 5b in the back-side portion 13 are laminated together. Meanwhile, in the C, for example, the breaking strength of the sample prepared by laminating two sheets of the material for the cover sheet 5 corresponds to that of the (two) cover sheets 5b and 5a in the stomach-side portion 11, or that of the (two) cover sheet 5a and 5b in the back-side portion 13 in Fig. 7. In addition, in the case of comparing the breaking strength in the fused portion 30 and the breaking strength of any one (a portion not including the fused portion 30) of the cover sheet 5 in the stomach-side portion 11 and the cover sheet 5 in the back-side portion 13, the laminate of four sheets of the material for the cover sheet 5 in the B and the laminate of two sheets of the material for the cover sheet 5 in the stomach-side portion 11 or the back-side portion 13 in the C are compared with each other.

In addition, as a preferable mode of the present embodiment, furthermore, the breaking strength in the fused portion 30 may be 110% or more when the breaking strength of a portion not including the fused portion 30 in any one of the cover sheet 5 in the stomach-side portion 11 (stomach-side sheet member) and the cover sheet 5 in the back-side portion 13 (back-side sheet member) is regarded as 100%. The methods of measuring the respective breaking strengths are as described in the section "B. breaking strength of fused portion" and the section "C. breaking strengths of cover sheets in stomach-side portion and back-side portion".

As described above, the disposable diaper 1 has the above-described breaking strength relationship, whereby, when the stomach-side portion 11 and the back-side portion 13 are pulled apart from each other, it is possible to make breakage more likely to occur at the boundary portion PB with the fused portion 30 in the cover sheet 5 (not including the fused portion) and to suppress the circumferential wall portions SW breaking at a larger number of the fused portions 30. Therefore, at the time of removing the absorbent article, it is possible to still more reliably separate the stomach-side portion 11 and the back-side portion 13 without breaking the cover sheet 5 in a portion excluding the joining portion 14a. It should be noted that there is no particular upper limit of the breaking strength of the fused portion 30. However, when the breaking strength is too high, the fused portion 30 becomes too stiff, and there is a concern that discomfort may be generated when a wearer or a caregiver comes into contact with the fused portions 30, and thus the upper limit is preferably 300% or less.

In addition, in the present embodiment, as another preferable mode, the dimension DT of the circumferential wall portion SW in the thickness direction T may be larger than the dimension dT of the core portion MC in the thickness direction T, and the position of the core portion MC in the thickness direction T in each of the plurality of fused portions 30 may be shifted toward the side of the stomach-side portion 11 from the boundary QB between the stomach-side portion 11 and the back-side portion 13 in the thickness direction T. In another embodiment, the position of the core portion MC may be shifted toward the side of the back-side portion 13 from the boundary QB.

As described above, in the disposable diaper 1, the positions of the core portions MC are shifted toward the side of the stomach-side portion 11 (shifted toward the side of the back-side portion 13 in another embodiment) from the boundary QB between the stomach-side portion 11 and the back-side portion 13 in all of the plurality of fused portions 30 in the joining portion 14a. Here, at the time of pulling apart the joining portion 14a, the fused portion 30 remains in the cover sheet 5 (sheet member) where the core portion MC is present, and a hole is formed due to the removal of the fused portion 30 in the cover sheet 5 (sheet member) where the core portion MC is not present (Fig. 10(a)). Therefore, in the disposable diaper 1, the positions of the core portions MC are shifted toward a specific side, that is, toward the side of the stomach-side portion 11 (shifted toward the side of the back-side portion 13 in another embodiment) in all of the plurality of fused portions 30 in the joining portion 14a. Therefore, it is possible to leave the fused portion 30 and form the hole on the same side, that is, on the side of the stomach-side portion 11 (on the side of the back-side portion 13 in another embodiment). Therefore, it is possible to more smoothly and appropriately separate the stomach-side portion 11 and the back-side portion 13 at the time of pulling apart the joining portion 14a.

In addition, in the present embodiment, as a preferable mode, the plurality of elastic members 8a (in the case of the stomach-side portion 11) and 8b (in the case of the back-side portion 13) that extend up to the joining portion 14a in the lateral direction W and are aligned at gaps in the longitudinal direction L may be further provided in at least one of the cover sheet 5 in the stomach-side portion 11 (stomach-side sheet member) and the cover sheet 5 in the back-side portion 13 (back-side sheet member). In addition, the plurality of elastic members 8a (in the case of the stomach-side portion 11) and 8b (in the case of the back-side portion 13) may not overlap the plurality of fused portions 30 in the thickness directions T in the joining portion 14a.

As described above, since the disposable diaper 1 has the above-described configuration, it is possible to avoid a situation where the elastic members 8a and 8b overlap the fused portions 30, the dimensions (thicknesses) of the circumferential wall portions SW in the in-plane direction become uneven, and the circumferential wall portion SW is likely to break. Therefore, at the time of removing the disposable diaper 1, the elastic members 8a and 8b disposed in the cover sheet 5 (sheet member) have no influence, and it is possible to suppress the circumferential wall portions SW breaking at a larger number of the fused portions 30 and to make breakage more likely to occur at the boundary portion PB with the fused portion 30 in the cover sheet 5. In addition, it is possible to suppress a situation where, at the time of removing the disposable diaper 1, the elastic members 8a and 8b are separated together with the fused portions 30 and come into contact with the skin of a wearer or caregiver in the middle of removing the disposable diaper 1. Therefore, it is possible to facilitate the more appropriate separation of the joining portion 14a into the stomach-side portion 11 and the back-side portion 13 without breaking the disposable diaper 1 in a portion excluding the joining portion 14a. It should be noted that the fact that the plurality of elastic members 8a (or 8b) do not overlap the plurality of fused portions 30 means that at least 80% or more (more preferably 90% or more) of the plurality of elastic members 8a (or 8b) do not overlap the plurality of fused portions 30.

In the present embodiment, as a preferable mode, the plurality of fused portions 30 may include a fused portion in which the shape of the core portion MC (in a plan view) as seen in the thickness direction T is circular or elliptical. As described above, since the disposable diaper 1 has the above-described configuration, in a case where the shape of the core portion MC is circular or elliptical, the shape drawn by the outer edge of the circumferential wall portion SW also becomes substantially circular or elliptical. Therefore, at the time of removing the disposable diaper 1, the boundary portion PB with the fused portion 30 in the cover sheet 5 (sheet member) becomes a curved surface with respect to the direction of a force at the time of breaking the boundary portion PB. Therefore, it is possible to make breakage likely to gradually occur at the boundary portion PB with the fused portion 30 in the cover sheet 5 along the circular or elliptical curved surface in each of the fused portions 30. Therefore, it becomes possible to facilitate the smooth and appropriate separation of the stomach-side portion 11 and the back-side portion 13.

In addition, as a preferable mode of the present embodiment, the breaking strengths of the plurality of fused portions 30 disposed along the longitudinal direction L in the joining portion 14a may be the same as each other. As described above, since the disposable diaper 1 has the above-described configuration, at the time of removing the disposable diaper 1, a wearer or a caregiver is able to continuously and stably pull the joining portion 14a apart along the longitudinal direction L all the way to the end using a force of substantially the same magnitude. That is, it becomes possible to facilitate the smooth and appropriate separation of the stomach-side portion 11 and the back-side portion 13. Here, the expression "the same" mentioned herein means that all of the breaking strengths of the plurality of fused portions 30 are included in a range of the average value of the breaking strengths ± 20%.

Here, among the plurality of fused portions 30 disposed along the longitudinal direction L, the breaking strengths of at least the fused portions 30 in the central region 22 are preferably the same as each other. The reason therefor is as described below. When a wearer (or a caregiver) pulls apart the joining portion 14a from an end part of the joining portion 14a in the longitudinal direction L, for example, from the end part on the waist opening WO side, the wearer (or caregiver) pulls apart the joining portion 14a by pinching the end parts 11e and 13e of the joining portion 14a on the waist opening WO side. At this time, since the fused portions 30 in the waist-side end part region 21 are close to the position pinched by the wearer, a force for pulling apart the joining portion 14a is easily transmitted. Therefore, the fused portions 30 are relatively easily pulled apart. However, since the fused portions 30 in the central region 22 are far from the position pinched by the wearer, the force for pulling apart the joining portion 14a is not easily transmitted. Therefore, when the breaking strengths of the fused portions 30 are uneven and a fused portion 30 having a high breaking strength is positioned behind a fused portion 30 having a low breaking strength, it is necessary to abruptly increase the pulling force to pull apart the fused portion 30 having a high breaking strength, and there is a concern that the wearer may feel a difficulty in pulling apart the joining portion 14a. Therefore, the breaking strengths of at least the fused portions 30 in the central region 22 are made the same as each other, whereby it is possible to pull apart the joining portion 14a without letting the wearer feel the difficulty of pulling apart the joining portion 14a. This fact also applies in the same manner when the wearer (or caregiver) pulls apart the joining portion 14a from the end part on the leg opening LO side in the longitudinal direction L, that is, when the joining portion 14a is pulled apart by pinching the end parts 11e' and 13e' of the joining portion 14a on the leg opening LO side.

In addition, as a preferable mode of the present embodiment, in each of the plurality of fused portions 30, the area Sa of the circumferential wall portion SW may be in a range of 5% to 60% as seen in the thickness direction T when the area Sb of the core portion MC is regarded as 100%. As described above, in the disposable diaper 1, the lower limit of the area Sa of the circumferential wall portion SW is 5% of the area Sb of the core portion MC in the fused portion 30 as the reference. That is, while the main part that plays a function of joining the fused portion 30 is the core portion MC, the circumferential wall portion SW can be made stronger by increasing the proportion of the circumferential wall portion SW in the core portion MC. Therefore, it is possible to make the fused portion 30 strong (increase the breaking strength) compared with the cover sheet 5 (sheet member) and to make breakage likely to occur at the boundary portion PB with the fused portion 30 in the cover sheet 5. Therefore, at the time of removing the disposable diaper 1, it is possible to still more reliably separate the stomach-side portion 11 and the back-side portion 13 without breaking the cover sheet 5 in a portion excluding the joining portion 14a. It should be noted that the upper limit of the area Sa of the circumferential wall portion SW is preferably 60% or less from the viewpoint of not hindering the function of joining the core portion MC.

In addition, as another embodiment, in the fused portion line 30L, the shapes of the plurality of fused portions 30 in the central region 22 and in the leg-side end part region 23 may be different from the shapes of the plurality of fused portions 30 in the waist-side end part region 21.

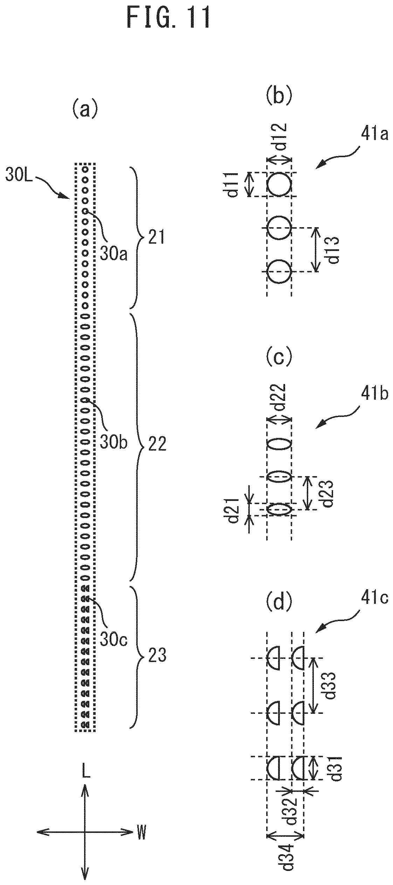

Fig. 11 is a plan view for describing another configuration example of the plurality of fused portions 30 in the joining portion 14a. Fig. 11(a) shows the fused portions 30a to 30c (fused portion line 30L) as seen from the side of the back-side portion 13 in the thickness direction T, and Fig. 11(b) shows the shapes and dispositions of the protruding portions 41a to 41c of the anvils in which the fused portions 30a to 30c are formed by the ultrasonic sealing method. In the present embodiment, the respective fused portions 30a, 30b, and 30c in the waist-side end part region 21, the central region 22, and the leg-side end part region 23 have mutually different planar shapes. Therefore, in the case of forming the fused portions 30a, 30b, and 30c by the ultrasonic sealing method, protruding portions 41a, 41b, and 41c of the anvils for the fused portions 30a, 30b, and 30c are different from each other.

The planar shape of the protruding portion 41a of the anvil for forming the fused portion 30a in the waist-side end part region 21 is a circular shape (truly circular shape), and a dimension d11 in the longitudinal direction L is equal to a dimension d12 in the lateral direction W. d11 and d12 are, for example, 0.5 mm to 5 mm. A distance d13 between the fused portions 30a adjacent to each other in the longitudinal direction L (the distance between the centers of the circles) is 1 mm to 30 mm.

The planar shape of the protruding portion 41c of the anvil for forming the fused portion 30c in the leg-side end part region 23 is a shape of a semicircle having a chord in the lateral direction W, and two protruding portions 41c are aligned close to each other in the lateral direction W. A dimension d31 in the longitudinal direction L, a dimension d32 in the lateral direction W, and a dimension d34 between the front end part of one protruding portion 41c and the back end part of the other protruding portions 41c aligned in the lateral direction W are 0.5 mm to 5 mm. A distance d33 between the fused portions 30c adjacent to each other in the longitudinal direction L (the distance between the centers of the circles) is 1 mm to 30 mm.

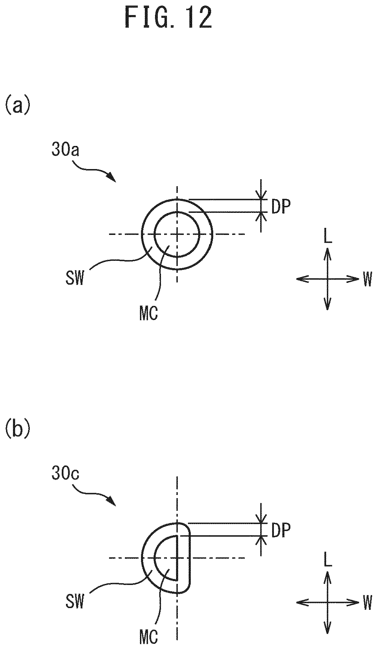

Fig. 12 is a plan view showing an example of the configuration of the fused portion 30 in the joining portion 14a. Here, Fig. 12(a) shows the fused portion 30a, and Fig. 12(b) shows the fused portion 30c. The fused portion 30b is the same as the fused portion 30 in Fig. 7. Each of the fused portions 30a and 30c includes the core portion MC and the circumferential wall portion SW. The core portion MC and the circumferential wall portion SW are the same as the core portion MC and the circumferential wall portion SW of the fused portion 30 in Fig. 7 except that the shapes thereof in a plan view are different.

In addition, it is not necessary that all of the fused portions in the joining portion 14a have the configurations of the fused portion 30 or the fused portions 30a to 30c as described above. For example, only the fused portions in a portion that covers at least 30% of the joining portion 14a in the longitudinal direction L from the end edges 11ae and 13ae, which are portions on the side of the end parts 11e and 13e on the waist opening WO side where the joining portion 14a begins to be pulled apart (begins to break), may have the above-described configuration. Similarly, only the fused portions in a portion that covers at least 30% of the joining portion 14a in the longitudinal direction L from the end edges 11ae' and 13ae', which are portions on the side of the end parts 11e' and 13e' on the leg opening LO side where the joining portion 14a begins to be pulled apart (begins to break), may have the above-described configuration. In these cases, it is possible to easily begin to pull apart the joining portion 14a.

EXAMPLES

Hereinafter, the present invention will be more specifically described by exemplifying examples and comparative examples, but the present invention is not limited only to such examples.

1. Regarding evenness of circumferential wall portions

(1) Sample

Example 1: A disposable diaper shown in Fig. 1 to Fig. 7 was prepared. The sealing method described in Patent Literature 2, which has been described in the description of Fig. 4, was used. The contact time during which the cover sheet 5 was in contact with the ultrasonic horn 43 and the protruding portion 41 was set to approximately 0.05 seconds, the total area of the plurality of protruding portions 41 for the joining portion 14a was set to approximately 110 mm$^2$, and the size of one protruding portion 41 was set to approximately 1.5 mm$^2$. The intensity of an ultrasonic wave (30 kHz) that was supplied by the ultrasonic horn 43 was set to substantially the same as that in the case of Comparative Example 1. Here, as each of the cover sheet 5 in the stomach-side portion 11 and the cover sheet 5 in the back-side portion 13, 15 g/m$^2$ of an SMS nonwoven fabric was used. Comparative Example 1: A disposable diaper having the same structure as the disposable diaper of Example 1 was prepared using the sealing method described in Patent Literature 1. The shape of the protruding portion of the anvil was set to an elliptical shape as in Fig. 7(a), and a joining portion was formed. The contact time during which the cover sheet was in contact with the ultrasonic horn and the protruding portion was set to approximately 0.005 seconds. The total area of the plurality of protruding portions for the joining portion was set to approximately 180 mm$^2$, and the size of one protruding portion was set to approximately the same as in Example 1. The intensity of an ultrasonic wave (30 kHz) that was supplied by the ultrasonic horn was set to substantially the same as that in the case of Example 1. As each of the cover sheet in the stomach-side portion and the cover sheet in the back-side portion, 15 g/m$^2$ of an SMS nonwoven fabric was used.

(2) Peel test of joining portion

In the disposable diaper of Example 1, the section "A. peel test of joining portion" was performed on each of the joining portion 14a on the left side and the joining portion 14b on the right side in the lateral direction W when seen from the front. Similarly, in the disposable diaper of Comparative Example 1, the section "A peel test of joining portion" was performed on each of the joining portion on the left side and the joining portion on the right side in the lateral direction when seen from the front.

(3) Measurement of width of circumferential wall portion

In the disposable diaper of Example 1, the dimension (thickness) of the circumferential wall portion SW of the fused portion 30 was measured. Specifically, the width DP (W, L) and the width DP (W, R) of the portions of the circumferential wall portion SW that are on the left side and on the right side, respectively, in the lateral direction W of the core portion MC were measured by cutting the fused portion 30 on a cross section perpendicular to the longitudinal direction L that passed through the measurement positions of the widths DP and observing the fused portion 30 with a scanning electron microscope. Similarly, the width DP (L, L) and the width DP (L, R) of the circumferential wall portion SW that are on the left side and on the right side, respectively, in the longitudinal direction L of the core portion MC were measured by cutting the fused portion 30 on a cross section perpendicular to the lateral direction W that passed through the measurement positions of the widths DP and observing the fused portion 30 with the electron microscope. Similarly, for each of the disposable diaper of Comparative Example 1, the width DP (W, L) and the width DP (W, R) of the circumferential wall portion SW of the fused portion that are on the left side and on the right side, respectively, in the lateral direction W of the core portion MC and the width DP (L, L) and the width DP (L, R) of the circumferential wall portion SW that are on the left side and on the right side, respectively, in the longitudinal direction L of the core portion MC were measured.

(4) Evaluation results

The evaluation results are shown in Table 1 below. In the disposable diaper of Example 1, the ratios a/b of the area a of the short-gap side region Aa to the area b of the long-gap side region Ab in the specific region were $0.5 \leq a/b \leq 2.0$ in both of the right and left joining portions 14b and 14a. That is, the area a and the area b were substantially the same as each other. Therefore, in both of the right and left joining portions 14b and 14a, a place where the width became partially too small was not generated in the circumferential wall portion SW, and the dimension (width DP) in the in-plane direction in the circumferential wall portion SW was relatively even. It should be noted that the maximum values F1 of the loads F at that time (maximum load (F1) in Table 1) were 14.5 to 16.0 N/25 mm respectively and were approximately the same on the right and the left. It should be noted that the total number of the fused portions 30 was 60, six fused portions 30 were measured in a single measurement, and the above-described numerical range was satisfied in all of 10 measurements.

On the other hand, in the disposable diaper of Comparative Example 1, the ratios a/b of the area a of the short-gap side region Aa to the area b of the long-gap side region Ab in the specific region were $2.0 < a/b$ in both of the right and left joining portions 14b and 14a, and the area a and the area b were significantly different from each other. Therefore, in both of the right and left joining portions 14b and 14a, a place where the width was partially too small was generated in the circumferential wall portion SW, and the dimension (width DP) in the in-plane direction in the circumferential wall portion SW was not even. It should be noted that the maximum values F1 at that time (maximum load (F1)) were 15.0 to 17.1 N/25 mm respectively and were approximately the same on the right and the left. It should be noted that the total number of the fused portions was 60, six fused portions were measured in a single measurement, and the above-described numerical range was not satisfied in all of 10 measurements.

The width DP of the circumferential wall portion SW was actually measured, and the following results were obtained. In Example 1, in the right joining portion 14b, the difference Δ1 between the width DP (W, L) on the left side (L) and the width DP (W, R) on the right side (R) of the circumferential wall portion SW in the lateral direction W was 2.5% of the average value DP (W)ave. In addition, the difference Δ2 between the width DP (L, U) on the upper side (D) and the width DP (L, D) on the lower side (D) of the circumferential wall portion SW in the longitudinal direction L was 6.7% of the average value DP (L)ave. In addition, the ratio between the average value DP (L)ave and the average value DP (W)ave was 0.84, and it was possible to say that both average values were substantially the same as each other. From the above-described results, it was found that, in Example 1, the dimension (width DP) of the circumferential wall portion SW in the in-plane direction was relatively even in the right joining portion 14b. Similarly, in the left joining portion 14a, the difference Δ1 was 0.65% of the average value DP (W)ave. In addition, the difference Δ2 was 17% of the average value DP (L)ave. In addition, the ratio between the average value DP (L)ave and the average value DP (W)ave was 0.79, and it was possible to say that both average values were substantially the same as each other. From the above-described results, it was found that, in Example 1, the dimension (width DP) of the circumferential wall portion SW in the in-plane direction was also relatively even in the left joining portion 14a. Therefore, it was found that, in Example 1, the dimension (width DP) of the circumferential wall portion SW in the in-plane direction was relatively even.

On the other hand, in Comparative Example 1, in the right joining portion 14b, the difference Δ2 was 17% of the average value DP (L)ave, but the difference Δ1 was 94% of the average value DP (W)ave. In other words, DP (W, L) was approximately 1/3 of DP (W, R). That is, it was found that, in Comparative Example 1, a place where the width DP was uneven in the longitudinal direction L was present in the circumferential wall portion SW. That is, actually, it was found that the dimension (width DP) in the in-plane direction was uneven in the circumferential wall portion SW (it should be noted that measurement was not performed in the left joining portion 14a).

Here,

$$\Delta1/DP\ (W)ave\ (\%) = |DP\ (W, L) - DP\ (W, R)|/DP\ (W)ave \times 100$$

$$DP\ (W)ave = (DP\ (W, L) + DP\ (W, R))/2 \qquad \Delta2/DP\ (L)ave\ (\%) = |DP\ (L, U) - DP\ (L, D)|/DP\ (L)ave \times 100$$

$$DP\ (L)ave = (DP\ (L, U) + DP\ (L, D))/2.$$

[Table 1]

| Type of joining portion | Example 1 | | Comparative Example 1 | |
|---|---|---|---|---|
| | Right joining portion 14b | Left joining portion 14a | Right joining portion 14b | Left joining portion 14a |
| a | 6837 | 7188 | 4630 | 3698 |
| b | 4761 | 6332 | 1825 | 1734 |
| a/b | 1.44 | 1.14 | 2.54 | 2.13 |
| Maximum load (F1) | 14.5 | 16.0 | 15.0 | 17.1 |
| DP (W, L) | 165 | 152 | 59 | - |
| DP (W, R) | 159 | 153 | 163 | - |
| DP (W)ave | 162 | 153 | 110 | - |
| Δ1/DP (W)ave (%) | 2.5 | 0.65 | 94 | - |
| DP (L, U) | 139 | 137 | 110 | - |
| DP (L, D) | 130 | 116 | 94 | - |
| DP (L)ave | 134 | 121 | 94 | - |
| Δ2/DP (L)ave (%) | 6.7 | 17 | 17 | - |
| DP (L)ave /DP (W)ave | 0.83 | 0.79 | 0.85 | - |

2. Regarding breaking strengths of fused portion and cover sheet

(1) Sample

Example 2: A sample for measuring the breaking strength of the fused portion was produced by the method described in the (1) of the section "B. breaking strength of fused portion" using one nonwoven fabric for the cover sheet 5 in the stomach-side portion 11 and one nonwoven fabric for the cover sheet 5 in the back-side portion 13, that is, a total of two nonwoven fabrics. In addition, one nonwoven fabric for the cover sheet 5 in the stomach-side portion or the back-side portion was used as a sample for measuring the breaking strength of the cover sheet.

Example 3: A sample for measuring the breaking strength of the fused portion was produced by the method described in the (1) of the section "B. breaking strength of fused portion" using two nonwoven fabrics for the cover sheet 5 in the stomach-side portion 11 and two nonwoven fabrics for the cover sheet 5 in the back-side portion 13, that is, a total of four nonwoven fabrics. In addition, two nonwoven fabrics for the cover sheet 5 in the stomach-side portion or the back-side portion were used as a sample for measuring the breaking strength of the cover sheet. Comparative Example 2: As a sample for measuring the breaking strength of the fused portion, the sample for measuring the breaking strength of the fused portion of Example 2 was used, and, as a sample for measuring the breaking strength of the cover sheet, the sample for measuring the breaking strength of the cover sheet of Example 3 was used. In the sample for measuring the breaking strength of the fused portion, the number of the cover sheets, which was essentially supposed to be four, was decreased to two, thereby decreasing the number of materials that constituted the fused portion and simulating the fact that the fused portion was not even.

(2) Measurement of breaking strength

For the samples for measuring the breaking strength of the fused portion of Examples 2 and 3 and Comparative Example 2, the breaking strengths were measured based on the section "B. breaking strength of fused portion". For the samples for measuring the breaking strength of the cover sheet of Examples 2 and 3 and Comparative Example 2, the breaking strengths were measured based on the section "C. breaking strengths of cover sheets in stomach-side portion and back-side portion".

(3) Evaluation results

The evaluation results are shown in Table 2 below. In Example 2, the breaking strength K2 of the fused portion including two nonwoven fabrics, which simulated a joining portion including one cover sheet in the stomach-side portion and one cover sheet in the back-side portion, was higher than the breaking strength K1 of one nonwoven fabric, which simulated one cover sheet in the stomach-side portion or the back-side portion (K1 < K2). Similarly, in Example 3, the breaking strength K2 of the fused portion including four nonwoven fabrics, which simulated a joining portion including two cover sheets in the stomach-side portion and two cover sheets in the back-side portion, was higher than the breaking strength K1 of two nonwoven fabrics, which simulated two cover sheets in the stomach-side portion or the back-side portion (K1 < K2). On the other hand, in Comparative Example 2, the breaking strength K2 of a fused portion including two nonwoven fabrics, which simulated a joining portion including one cover sheet in the stomach-side portion and one cover sheet in the back-side portion, that is, had a weaker breaking strength than the fused portion including four nonwoven fabrics, became smaller than the breaking strength K1 of two nonwoven fabrics, which simulated two cover sheets in the stomach-side portion or the back-side portion (K1 > K2). From these results, it is considered that, since the breaking strength of the fused portion is higher than the breaking strength of the cover sheet as long as a side wall portion that corresponds to the circumferential wall portion of the fused portion is not uneven, it becomes possible to cause breakage at the boundary portion with the fused portion in the cover sheet. Therefore, when it is possible to form the circumferential wall portion substantially evenly regardless of the shape of the fused portion, it is possible to converge the breaking strength necessary for pulling apart the joining portion to the breaking strength of the cover sheet. Therefore, it is possible to control the pulling of the joining portion with the breaking strength of the material (cover sheet), and thus it becomes possible to stably pull apart the joining portion by appropriately setting the breaking strength of the material.

[Table 2]

| | Nonwoven fabric alone | | Fused portion including nonwoven fabrics | | Relationship between K1 and K2 |
| --- | --- | --- | --- | --- | --- |
| | Number of sheets | Breaking strength K1 (N/5 mm) | Number of sheets | Breaking strength K2 (N/5 mm) | |
| Example 2 | 1 | 2.51 | 2 | 3.16 | K1<K2 |
| Example 3 | 2 | 4.82 | 4 | 8.01 | K1<K2 |
| Comparative Example 2 | 2 | 4.82 | 2 | 3.16 | K1>K2 |

The disposable diaper of the present invention is not limited to each of the above-described embodiments and can be appropriately combined, modified, or the like without departing from the subject matter of the present invention. That is, a variety of techniques described in each embodiment are also applicable to other embodiments as long as the techniques create no contradiction.

REFERENCE SIGNS LIST

1 Disposable diaper
11 Stomach-side portion
13 Back-side portion
14a Joining portion
30 Fused portion
MC Core portion
SW Circumferential wall portion

**Claims**

1. An absorbent article having a longitudinal direction (L), a lateral direction (W), and a thickness direction (T) that are orthogonal to each other, the absorbent article comprising:
a stomach-side portion (11) including a stomach-side sheet member; and a back-side portion (13) including a back-side sheet member, wherein the absorbent article further comprises a joining portion (14a, 14b) in which at least one end part (11a, 11b) of the stomach-side portion (11) and at least one end part (13a, 13b) of the back-side portion (13) in the lateral direction (W) are joined to each other along the longitudinal direction (L) in a state of overlapping each other in the thickness direction (T),

wherein the joining portion (14a, 14b) includes a plurality of fused portions (30) disposed at gaps along the longitudinal direction (L),
wherein each of the plurality of fused portions (30) further includes:

a core portion (MC) in which the stomach-side sheet member and the back-side sheet member are fused to each other in the thickness direction (T), and
a circumferential wall portion (SW) in which the stomach-side sheet member and the back-side sheet member are fused to each other in the thickness direction (T) in a way such that the circumferential wall portion (SW) extends from a circumferential edge of the core portion (MC) in the thickness direction (T) while surrounding the core portion (MC) in a tubular shape,
wherein, in a T-type peel test of the joining portion (14a, 14b), in which a sample including a part of the joining portion (14a, 14b) is gripped with two jigs, the joining portion (14a, 14b) is peeled off such that a gap (D) between the two jigs broadens, and a load (F) that is applied between the two jigs is measured, in a region indicated by a graph (G) showing the load (F) with respect to the gap (D), when a specific region, in which the load (F) is equal to or greater than a predetermined reference load (F0), is divided into a short-gap side region (Aa) and a long-gap side region (Ab) with respect to a maximum load gap (D1) as a boundary, the load of which being a maximum value (F1), a ratio between an area a of the short-gap side region (Aa) and an area b of the long-gap side region (Ab), which is represented by a/b, is 0.5 or more and 2.0 or less, measured as described in the description, and
wherein breaking strengths in the fused portions (30) are higher than breaking strengths of portions not including the fused portions (30) in any one of the stomach-side sheet member and the back-side sheet member.

2. The absorbent article according to claim 1,
wherein, when the breaking strengths of the portions not including the fused portions (30) in any one of the stomach-side sheet member and the back-side sheet member are regarded as 100%, the breaking strengths in the fused portions (30) are 110% or higher.

3. The absorbent article according to claim 1 or claim 2,
wherein a dimension of the circumferential wall portion (SW) in the thickness direction (T) is larger than a dimension of the core portion (MC) in the thickness direction (T), and
a position of the core portion (MC) in the thickness direction (T) in each of the plurality of fused portions (30) is shifted toward a side of the stomach-side portion (11) or a side of the back-side portion (13) from a boundary between the stomach-side portion (11) and the back-side portion (13) in the thickness direction (T).

4. The absorbent article according to any one of claims 1 to 3, further comprising:

a plurality of elastic members (8a, 8b) that extend up to the joining portion (14a, 14b) in the lateral direction (W) and are aligned at gaps in the longitudinal direction (L) in at least one of the stomach-side sheet member and the back-side sheet member,
wherein the plurality of elastic members (8a, 8b) do not overlap the plurality of fused portions (30) in the thickness direction (T) in the joining portion (14a, 14b).

5. The absorbent article according to any one of claims 1 to 4,
wherein the plurality of fused portions (30) include a fused portion in which the core portion (MC) has a circular or elliptical shape as seen in the thickness direction (T).

6. The absorbent article according to any one of claims 1 to 5,
wherein the plurality of fused portions (30) disposed along the longitudinal direction (L) in the joining portion (14a, 14b) have the same breaking strength.

7. The absorbent article according to any one of claims 1 to 6,
wherein, in each of the plurality of fused portions (30), when an area of the core portion (MC) is regarded as 100%, an area of the circumferential wall portion (SW) is in a range of 5% to 60% as seen in the thickness direction (T).


**Patentansprüche**

1. Absorbierender Artikel, der eine Längsrichtung (L), eine seitliche Richtung (W) und eine Dickenrichtung (T) aufweist, die senkrecht zueinander sind, wobei der absorbierende Artikel Folgendes umfasst:
einen Bauchseitenteil (11), der ein Bauchseitenlagenelement einschließt, und einen Rückenseitenteil (13), der ein Rückenseitenlagenelement einschließt; wobei der absorbierende Artikel weiter einen Verbindungsteil (14a, 14b) umfasst, in dem mindestens ein Endteil (11a, 11b) des Bauchseitenteils (11) und mindestens ein Endteil (13a, 13b) des Rückenseitenteils (13) in der seitlichen Richtung (W) entlang der Längsrichtung (L) in einem Zustand, in dem sie einander in der Dickenrichtung (T) überlappen, miteinander verbunden sind,

wobei der Verbindungsteil (14a, 14b) eine Vielzahl von verschweißten Teilen (30) einschließt, die in Abständen entlang der Längsrichtung (L) angeordnet sind,
wobei jeder der Vielzahl von verschweißten Teilen (30) weiter Folgendes einschließt:

einen Kernteil (MC), in dem das Bauchseitenlagenelement und das Rückenseitenlagenelement in der Dickenrichtung (T) miteinander verschweißt sind, und
einen umlaufenden Wandteil (SW), in dem das Bauchseitenlagenelement und das Rückenseitenlagenelement in der Dickenrichtung (T) auf eine Weise miteinander verschweißt sind, dass sich der umlaufende Wandteil (SW) von einem umlaufenden Rand des Kernteils (MC) in der Dickenrichtung (T) erstreckt, während er den Kernteil (MC) in einer röhrenförmigen Form umgibt,
wobei in einem T-Peel-Test des Verbindungsteils (14a, 14b), in dem eine Probe, die einen Teil des Verbindungsteils (14a, 14b) einschließt, mit zwei Vorrichtungen gegriffen wird, der Verbindungsteil (14a, 14b) derart abgezogen wird, dass sich ein Abstand (D) zwischen den zwei Vorrichtungen verbreitert, und eine Beanspruchung (F), die zwischen den zwei Vorrichtungen angelegt wird, gemessen wird,
in einem Bereich, der durch eine Kurve (G), die die Beanspruchung (F) in Bezug auf den Abstand (D) zeigt, angezeigt wird, wenn ein spezifischer Bereich, in dem die Beanspruchung (F) gleich oder größer ist als eine vorgegebene Referenzbeanspruchung (F0), in einen Kurzabstandsseitenbereich (Aa) und einen Langabstandsseitenbereich (Ab) in Bezug auf einen maximalen Beanspruchungsabstand (D1) als eine Grenze geteilt wird, deren Beanspruchung ein maximaler Wert (F 1) ist, ein Verhältnis zwischen einer Fläche a des Kurzabstandsseitenbereichs (Aa) und einer Fläche b des Langabstandsseitenbereichs (Ab), das durch a/b dargestellt wird, 0,5 oder mehr und 2,0 oder weniger beträgt, was wie in der Beschreibung beschrieben gemessen wird, und
wobei Bruchfestigkeiten in den verschweißten Teilen (30) höher sind als Bruchfestigkeiten von Teilen, die nicht die verschweißten Teile (30) in einem des Bauchseitenlagenelements und des Rückenseitenlagenelements einschließen.

2. Absorbierender Artikel nach Anspruch 1,
wobei, wenn die Bruchfestigkeiten der Teile, die nicht die verschweißten Teile (30) in einem des Bauchseitenlagenelements und des Rückenseitenlagenelements einschließen, als 100 % betrachtet werden, die Bruchfestigkeiten in den verschweißten Teilen (30) 110 % oder höher sind.

3. Absorbierender Artikel nach Anspruch 1 oder Anspruch 2,
wobei eine Abmessung des umlaufenden Wandteils (SW) in der Dickenrichtung (T) größer ist als eine Abmessung des Kernteils (MC) in der Dickenrichtung (T) und eine Position des Kernteils (MC) in der Dickenrichtung (T) in jedem der Vielzahl von verschweißten Teilen (30) in Richtung einer Seite des Bauchseitenteils (11) oder einer Seite des Rückenseitenteils (13) von einer Grenze zwischen dem Bauchseitenteil (11) und dem Rückenseitenteil (13) in der Dickenrichtung (T) verschoben ist.

4. Absorbierender Artikel nach einem der Ansprüche 1 bis 3, der weiter Folgendes umfasst:

eine Vielzahl von elastischen Elementen (8a, 8b), die sich bis zu dem Verbindungsteil (14a, 14b) in der seitlichen Richtung (W) erstrecken und in Abständen in der Längsrichtung (L) in mindestens einem des Bauchseitenlagenelements und des Rückenseitenlagenelements ausgerichtet sind,
wobei die Vielzahl von elastischen Elementen (8a, 8b) nicht mit der Vielzahl von verschweißten Teilen (30) in der Dickenrichtung (T) in dem Verbindungsteil (14a, 14b) überlappt.

**5.** Absorbierender Artikel nach einem der Ansprüche 1 bis 4,
wobei die Vielzahl von verschweißten Teilen (30) einen verschweißten Teil einschließt, in dem der Kernteil (MC) eine kreisförmige oder elliptische Form aufweist, wie in der Dickenrichtung (T) gesehen wird.

**6.** Absorbierender Artikel nach einem der Ansprüche 1 bis 5,
wobei die Vielzahl von verschweißten Teilen (30), die entlang der Längsrichtung (L) in dem Verbindungsteil (14a, 14b) angeordnet ist, die gleiche Bruchfestigkeit aufweist.

**7.** Absorbierender Artikel nach einem der Ansprüche 1 bis 6,
wobei in jedem der Vielzahl von verschweißten Teilen (30), wenn eine Fläche des Kernteils (MC) als 100 % betrachtet wird, eine Fläche des umlaufenden Wandteils (SW) in einem Bereich von 5 % bis 60 % liegt, wie in der Dickenrichtung (T) gesehen wird.

**Revendications**

**1.** Article absorbant ayant une direction allant dans le sens longitudinal (L), une direction allant dans le sens latéral (W) et une direction allant dans le sens de l'épaisseur (T) qui sont orthogonales les unes par rapport aux autres, l'article absorbant comportant :
une partie côté estomac (11) comprenant un élément de feuille côté estomac ; et une partie côté dos (13) comprenant un élément de feuille côté dos, dans lequel l'article absorbant comporte par ailleurs une partie d'assemblage (14a, 14b) dans laquelle au moins une partie d'extrémité (11a, 11b) de la partie côté estomac (11) et au moins une partie d'extrémité (13a, 13b) de la partie côté dos (13) dans la direction allant dans le sens latéral (W) sont assemblées l'une à l'autre le long de la direction allant dans le sens longitudinal (L) dans un état de chevauchement l'une par rapport à l'autre dans la direction allant dans le sens de l'épaisseur (T),

dans lequel la partie d'assemblage (14a, 14b) comprend une pluralité de parties fusionnées (30) disposées au niveau d'espaces le long de la direction allant dans le sens longitudinal (L),
dans lequel chacune de la pluralité de parties fusionnées (30) comprend par ailleurs :

une partie centrale (MC) dans laquelle l'élément de feuille côté estomac et l'élément de feuille côté dos sont fusionnés l'un par rapport à l'autre dans la direction allant dans le sens de l'épaisseur (T), et
une partie de paroi circonférentielle (SW) dans laquelle l'élément de feuille côté estomac et l'élément de feuille côté dos sont fusionnés l'un par rapport à l'autre dans la direction allant dans le sens de l'épaisseur (T) d'une telle manière que la partie de paroi circonférentielle (SW) s'étend depuis un bord circonférentiel de la partie centrale (MC) dans la direction allant dans le sens de l'épaisseur (T) tout en entourant la partie centrale (MC) en une forme tubulaire,
dans lequel, dans un essai de résistance au pelage de type T de la partie d'assemblage (14a, 14b), dans lequel un échantillon comprenant une partie de la partie d'assemblage (14a, 14b) est saisi au moyen de deux gabarits, la partie d'assemblage (14a, 14b) est pelée de telle sorte qu'un espace (D) entre les deux gabarits s'élargit, et une charge (F) qui est appliquée entre les deux gabarits est mesurée,
dans une région indiquée par un graphe (G) illustrant la charge (F) par rapport à l'espace (D), quand une région spécifique, dans laquelle la charge (F) est égale ou supérieure à une charge de référence prédéterminée (F0), est divisée en une région côté espace court (Aa) et une région côté espace long (Ab) par rapport à un espace de charge maximum (D1) comme limite, la charge de celle-ci étant une valeur maximum (F1), un rapport entre une zone a de la région côté espace court (Aa) et une zone b de la région côté espace long (Ab), qui est représenté par a/b, est de 0,5 ou plus et de 2,0 ou moins, la mesure étant telle qu'elle est décrite dans la description, et
dans lequel les résistances à la rupture dans les parties fusionnées (30) sont supérieures aux résistances à la rupture des parties ne comprenant pas les parties fusionnées (30) dans l'un quelconque parmi l'élément de feuille côté estomac et l'élément de feuille côté dos.

**2.** Article absorbant selon la revendication 1,
dans lequel, quand les résistances à la rupture des parties ne comprenant pas les parties fusionnées (30) dans l'un quelconque parmi l'élément de feuille côté estomac et l'élément de feuille côté dos sont considérées comme étant de 100 %, les résistances à la rupture dans les parties fusionnées (30) sont de 110 % ou plus.

**3.** Article absorbant selon la revendication 1 ou la revendication 2,

EP 3 821 862 B1

dans lequel une dimension de la partie de paroi circonférentielle (SW) dans la direction allant dans le sens de l'épaisseur (T) est supérieure à une dimension de la partie centrale (MC) dans la direction allant dans le sens de l'épaisseur (T), et une position de la partie centrale (MC) dans la direction allant dans le sens de l'épaisseur (T) dans chacune de la pluralité de parties fusionnées (30) est décalée vers un côté de la partie côté estomac (11) ou un côté de la partie côté dos (13) à partir d'une limite entre la partie côté estomac (11) et la partie côté dos (13) dans la direction allant dans le sens de l'épaisseur (T).

4. Article absorbant selon l'une quelconque des revendications 1 à 3, comportant par ailleurs :

une pluralité d'éléments élastiques (8a, 8b) qui s'étendent jusqu'à la partie d'assemblage (14a, 14b) dans la direction allant dans le sens latéral (W) et qui sont alignés au niveau des espaces dans la direction allant dans le sens longitudinal (L) dans au moins l'un parmi l'élément de feuille côté estomac et l'élément de feuille côté dos, dans lequel les éléments élastiques de la pluralité d'éléments élastiques (8a, 8b) ne chevauchent pas la pluralité de parties fusionnées (30) dans la direction allant dans le sens de l'épaisseur (T) dans la partie d'assemblage (14a, 14b).

5. Article absorbant selon l'une quelconque des revendications 1 à 4, dans lequel les parties fusionnées de la pluralité de parties fusionnées (30) comprennent une partie fusionnée dans laquelle la partie centrale (MC) a une forme circulaire ou elliptique tel que l'on peut voir dans la direction allant dans le sens de l'épaisseur (T).

6. Article absorbant selon l'une quelconque des revendications 1 à 5, dans lequel les parties fusionnées de la pluralité de parties fusionnées (30) disposés le long de la direction allant dans le sens longitudinal (L) dans la partie d'assemblage (14a, 14b) ont la même résistance à la rupture.

7. Article absorbant selon l'une quelconque des revendications 1 à 6, dans lequel, dans chacune de la pluralité de parties fusionnées (30), quand une zone de la partie centrale (MC) est considérée comme étant de 100 %, une zone de la partie de paroi circonférentielle (SW) se trouve dans une plage allant de 5 % à 60 % tel que l'on peut voir dans la direction allant dans le sens de l'épaisseur (T).

FIG. 1

# FIG. 2

# FIG. 3

# FIG. 4

# FIG. 5

# FIG. 6

(a)                    (b)

# FIG. 7

(a)

(b)

# FIG. 8

# FIG. 9

# FIG. 10

(a)

(b)

# FIG. 11

(a)

(b)

(c)

(d)

# FIG. 12

(a)

(b)

**EP 3 821 862 B1**

**REFERENCES CITED IN THE DESCRIPTION**

**Patent documents cited in the description**

- JP 2004298413 A

- JP 2006192902 A